# EUROPEAN PATENT APPLICATION

(11) **EP 1 652 915 A1**
(43) Date of publication of application: **03.05.2006**
(21) Application number: 04771467.0
(22) Date of filing: 10.08.2004
(51) Int. Cl.: C12N 15/00, C07K 14/195, C12N 9/16

(54) **METHODS OF DEGRADING dsRNA AND SYNTHESIZING RNA**

(30) Priority: 14.08.2003 JP 2003293553; 30.09.2003 JP 2003342126; 08.12.2003 JP 2003409639; 24.03.2004 JP 2004086129
(71) Applicant: TAKARA BIO INC., Otsu-shi, Shiga 520-2193 (JP)
(72) Inventor: Sagawa, Hiroaki, c/o Takara Bio Inc., Otsu-shi, Shiga 5202193 (JP); Tomono, Jun, Kurashiki-shi, Okayama 7100845 (JP); Ueno, Harumi, c/o Takara Bio Inc., Otsu-shi, Shiga 5202193 (JP); Kato, Ikunoshin, c/o Takara Bio Inc., Otsu-shi, Shiga 5202193 (JP)
(74) Representative: Schnappauf, Georg
(86) International application number: PCT/JP2004/011480
(87) International publication number: WO 2005/017144

(57) **Abstract**

A protein having an activity of degrading a dsRNA, namely, being capable of acting on a long-chain dsRNA to form a dsRNA of a definite length; a method of efficiently preparing a dsRNA of a definite length which comprises treating a dsRNA with the protein having an activity of degrading a dsRNA in the coexistence of a protein having an activity of binding to a nucleic acid such as a protein having an RNA-binding activity; and a method of using the protein having an activity of binding to a nucleic acid to elevate the efficiency in an RNA synthesis reaction typified by dsRNA synthesis.

## Description

### Technical Field

The present invention relates to a protein having an activity of degrading a dsRNA which has an activity of generating a dsRNA of a specific length, a method for efficiently degrading a dsRNA using said protein and a protein having an activity of binding to a nucleic acid (e.g., a protein having an activity of binding to an RNA) in combination, as well as a method for efficiently synthesizing an RNA using a protein having an activity of binding to a nucleic acid and a protein having an activity of synthesizing an RNA in combination.

### Background Art

Recently, genetic engineering techniques that utilize small dsRNAs have been reported.
For example, RNA interference (RNAi) is a phenomenon in which an mRNA is degraded with a dsRNA in a sequence-specific manner, resulting in suppression of gene expression. The beginning of the finding that a dsRNA can be used for gene silencing was a study in which an antisense was used in *Caenorhabditis elegans.* In 1995, Guo and Kemphues carried out an experiment to suppress a gene called par-1 using an antisense RNA. When an antisense RNA was added, the expression of par-1 was suppressed as expected. Surprisingly, a sense RNA which was used as a control also suppressed the expression of par-1, resulting in a phenotype of a par-1 mutant (see, for example, Non-patent Document 1).
This contradiction was resolved by Fire et al. in 1998. When an antisense RNA or a sense RNA is synthesized using an RNA polymerase, a small amount of an inverse RNA is unintentionally produced in a nonspecific manner. It has been shown that a dsRNA formed due to the contamination is the essential entity for the gene silencing, an antisense RNA or a sense RNA cannot suppress gene expression, and a dsRNA formed by annealing an antisense RNA to a sense RNA can efficiently suppress gene expression (e.g., Non-patent Document 2).

In RNA interference, an enzyme called a Dicer produces a small RNA (a short interfering RNA (siRNA)) from a dsRNA (e.g., Non-patent Document 3).
It is considered that the siRNA produced as a result of the action of the enzyme is incorporated into a complex called an RNA induced silencing complex (RISC), and the complex recognizes and degrades a target mRNA. However, the exact functions of the respective factors supposed to be involved in RNA interference have been unknown in many cases (e.g., Non-patent Document 4).

It is importance to efficiently produce a dsRNA or an siRNA in order to efficiently carry out RNA interference. The Dicer is exemplified by human Dicer (e.g., Non-patent Document 5). A recombinant Dicer (e.g., Non-patent Document 6) is sold by Gene Therapy Systems or Stratagene.
However, it has not been examined in detail whether or not the inherent enzymatic properties of the above-mentioned recombinant Dicer are sufficiently exhibited. Furthermore, the minimal domain of the Dicer to be included for retaining its activity of producing a preferable dsRNA (siRNA) upon its action on a dsRNA, or for increasing the productivity thereof using genetic engineering techniques is not known well.
In addition, a particularly effective method for efficient production of a dsRNA has not been known.

A method in which a dsRNA of about 21 nucleotides in length synthesized using an RNA polymerase is utilized as it is as an siRNA has been reported (e.g., Non-patent Document 7). A method by which an RNA can be efficiently produced can be utilized for the above-mentioned method, if it is available.

Non-patent Document 1: Guo, S. et al., Cell, 1995, vol.81, p.611-620
Non-patent Document 2: Fire, A. et al., Nature, 1998, vol.39, p.806-811
Non-patent Document 3: Bernstein, E. et al., Nature, 2001, vol.409, p.363-366
Non-patent Document 4: Tabara, H. et al., Cell, 2002, vol.109, p.861-871
Non-patent Document 5: Zhang, H. et al., The EMBO Journal, 2002, vol.21, No.21, p.5875-5885
Non-patent Document 6: Myers, J.W. et al., Nature Biotechnology, 2003, vol.21, p.324-328
Non-patent Document 7: Donze, O. et al., Nucleic Acids Research, 2002, vol.30, No.10, e46

### Disclosure of Invention

### Problems to be Solved by the Invention

The problems to be solved by the present invention are to provide a protein having an activity of degrading a dsRNA which has an activity of producing a dsRNA of a specific length, as well as to provide a method for efficiently producing a dsRNA of a specific length which can be utilized for RNA interference or the like, and a method for promoting RNA synthesis.

### Means to Solve the Problems

The present inventors have studied intensively in order to solve the above-mentioned problems. As a result, the present inventors have found, by analyzing a functional domain of a Dicer, a protein having an activity of degrading a dsRNA which has an activity of acting on a long dsRNA to produce a dsRNA of a specific length. Furthermore, the present inventors have found that a dsRNA of a specific length can be efficiently prepared by allowing a protein having an activity of degrading a dsRNA to act on a dsRNA in the presence of a protein having an activity of binding to a nucleic acid (e.g., a protein having an activity of binding to an RNA), and that the protein having an activity of binding to a nucleic acid also increases the efficiency of a reaction of RNA synthesis, the representative of which is dsRNA synthesis. Thus, the present invention has been completed.

The first aspect of the present invention relates to a protein having an activity of degrading a dsRNA, which has an activity of acting on a dsRNA to produce a dsRNA of a specific length.
According to the first aspect, the protein having an activity of degrading a dsRNA preferably has a functional domain of a Dicer. For example, it preferably consists of RNase IIIa, RNase IIIb and dsRNA-binding domains. The protein may further contain a PAZ domain. It is possible to produce a dsRNA of about 15 to 30 base pairs as the dsRNA of a specific length by using the protein of the first aspect. The protein having an activity of degrading a dsRNA of the first aspect is exemplified by a protein consisting of the amino acid sequence of SEQ ID NO:4 or 17, or an amino acid sequence encoded by the nucleotide sequence of SEQ ID NO:3 or 16. Alternatively, the protein may be a protein consisting of an amino acid sequence in which one or plural amino acid(s) is(are) substituted, deleted, inserted or added in the amino acid sequence of SEQ ID NO:4 or 17. The protein having an activity of degrading a dsRNA of the first aspect can be efficiently produced by converting a codon into one that is suitable for expression in a host, or by reinforcing a host to be used for a rare codon.
Furthermore, the protein can be expressed using a cold-inducible vector. In addition, the protein of the first aspect may be contained in a kit as a component.

The second aspect of the present invention relates to a method for degrading a dsRNA, the method comprising allowing a protein having an activity of degrading a dsRNA to act on a dsRNA in the presence of a protein having an activity of binding to a nucleic acid to produce a dsRNA of a specific length.
According to the second aspect, the protein having an activity of binding to a nucleic acid and the protein having an activity of degrading a dsRNA may be a fusion protein. The protein having an activity of binding to a nucleic acid may be a protein having an activity of binding to an RNA. The protein having an activity of binding to an RNA may be a cold shock protein. The cold shock protein may be derived from a thermophilic bacterium or a thermostable bacterium. Although it is not intended to limit the present invention, for example, the cold shock protein is exemplified by cold shock protein B from *Thermotoga maritima.*
According to the method of the second aspect, it is possible to produce a dsRNA of about 15 to 30 base pairs as the dsRNA of specific length. Furthermore, according to the second aspect, the protein having an activity of degrading a dsRNA may be the protein of the first aspect, or a native Dicer or a functional equivalent thereof.

The third aspect of the present invention relates to a method for synthesizing an RNA, the method comprising conducting an RNA synthesis reaction using a protein having an activity of synthesizing an RNA in the presence of a protein having an activity of binding to a nucleic acid.
According to the third aspect, the protein having an activity of binding to a nucleic acid and the protein having an activity of synthesizing an RNA may be a fusion protein. The protein having an activity of binding to a nucleic acid may be a cold shock protein. The cold shock protein may be derived from a thermophilic bacterium or a thermostable bacterium. Although it is not intended to limit the present invention, for example, the cold shock protein is exemplified by cold shock protein B from *Thermotoga maritima*. The protein having an activity of synthesizing an RNA may be a DNA-dependent RNA polymerase.

The fourth aspect of the present invention relates to a composition used for the method of the second aspect, the composition containing a protein having an activity of binding to a nucleic acid and a protein having an activity of degrading a dsRNA.

The fifth aspect of the present invention relates to a kit used for the method of the second aspect, the kit containing a protein having an activity of binding to a nucleic acid and a protein having an activity of degrading a dsRNA.

The sixth aspect of the present invention relates to a composition used for the method of the third aspect, the composition containing a protein having an activity of binding to a nucleic acid and a protein having an activity of synthesizing an RNA.

The seventh aspect of the present invention relates to a kit used for the method of the third aspect, the kit containing a protein having an activity of binding to a nucleic acid and a protein having an activity of synthesizing an RNA.

### Effects of the Invention

The present invention provides a protein having an activity of degrading a dsRNA which can be used to prepare a dsRNA of a specific length. Furthermore, a dsRNA of a specific length which can be utilized for RNA interference or the like can be efficiently produced according to the present invention.

### Best Mode for Carrying Out the Invention

As used herein, a Dicer refers to a protein that has a function by which a long dsRNA can be processed into an siRNA at an early stage of RNAi. Examples of native Dicers include, but are not limited to, one composed of, from the N terminus, an ATP-binding domain, an RNA helicase domain, a PAZ domain of unknown function, RNase IIIa and RNase IIIb domains, and a dsRNA-binding domain.

As used herein, a functional domain of a Dicer refers to a part that encodes a region involved in an activity by which a dsRNA of a specific length can be produced upon action on a long dsRNA.

Examples of the functional domains include, but are not limited to, one consisting of RNase IIIa, RNase IIIb and dsRNA-binding domains. The RNase IIIa and RNase IIIb domains may be a part that encodes a region involved in an activity of specifically acting on a double-stranded RNA to produce an oligonucleotide of a specific length having a phosphate group at the 5' end as described in Zhang, H. et al., The EMBO Journal, 2002, vol.21, No.21, p.5875-5885. The dsRNA-binding domain may be a part that encodes an activity of specifically binding to a double-stranded RNA.

As used herein, a dsRNA refers to an RNA forming a double-stranded structure between an mRNA to be subjected to RNA interference and an RNA having a nucleotide sequence complementary to the mRNA.
The main exemplary application of a product of a dsRNA degradation reaction according to the present invention is an siRNA as described below.

As used herein, a dsRNA of a specific length refers to a dsRNA of a specific length, for example, from about 10 to 100 base pairs although it is not intended to limit the present invention. It may be a dsRNA of a specific length from about 15 to 30 base pairs, particularly from 20 to 25 base pairs. Such a dsRNA can be used as an siRNA.

As used herein, a protein having an activity of binding to a nucleic acid refers to a protein having an activity of binding to a single-stranded or double-stranded DNA or RNA. The protein preferably has a function of canceling a secondary structure of a nucleic acid. Examples thereof include DNA helicases, RNA helicases and functional equivalents thereof.

As used herein, a cold-inducible vector refers to a vector having a promoter that is capable of functioning at a low temperature. Examples thereof include the pCold-series vectors as described in WO 99/27117.

As used herein, cold shock proteins generically refer to proteins expressed as a result of stimulation by lowered temperature as compared with the inherent growth conditions.

As used herein, complete degradation of a long dsRNA as a substrate refers to degradation to a degree that the uncleaved long dsRNA as a substrate is not observed after a degradation reaction as determined by electrophoresis.

As used herein, a PAZ domain is a domain between an RNA helicase domain and RNase IIIa and RNase IIIb domains in a Dicer. For example, it is a region from amino acid 898 to amino acid 1064 in the amino acid sequence of human Dicer of SEQ ID NO:1 (from nucleotide 2692 to nucleotide 3192 in SEQ ID NO:2) .

According to the present invention, a rare codon means a codon whose usage is low. A single amino acid may be prescribed by plural codons. The usages of the plural codons may be biased depending on the biological species. It is known that the amount of a transfer RNA (tRNA) corresponding to a codon of low usage is generally small, and expression efficiency is decreased if an amino acid sequence encoded by a nucleotide sequence that contains a rare codon is to be expressed. Thus, a protein can be efficiently produced by reinforcement for a rare codon (e.g., by increasing the amount of a tRNA corresponding to a rare codon).

Hereinafter, the present invention will be described in detail.
(1) Protein having an activity of degrading a dsRNA of the present invention, method for producing the protein, and kit containing the protein
The protein having an activity of degrading a dsRNA of the present invention can act on a dsRNA to produce a dsRNA of a specific length. There is no specific limitation concerning the protein having an activity of degrading a dsRNA as long as it can produce a dsRNA of a specific length from a long dsRNA. It is exemplified by a protein having a functional domain of a Dicer. The functional domain of a Dicer may be a protein consisting of RNase IIIa, RNase IIIb and dsRNA-binding domains. Any protein may be used regardless of its origin as long as it can produce a dsRNA of a specific length from a long dsRNA.
There is no specific limitation concerning the RNase IIIa, RNase IIIb and dsRNA-binding domains. In case of human Dicer, an exemplary one has amino acid 1271 to amino acid 1924 in the N-terminal portion of the amino acid sequence of SEQ ID NO:1 (nucleotide 3811 to nucleotide 5772 in the nucleotide sequence of SEQ ID NO:2). Examples thereof include a protein (a Dicer mutant) consisting of the amino acid sequence of SEQ ID NO:4 or an amino acid sequence encoded by the nucleotide sequence of SEQ ID NO:3, and a protein (a Dicer mutant) consisting of the amino acid sequence of SEQ ID NO:12 or an amino acid sequence encoded by the nucleotide sequence of SEQ ID NO:13.
Since the Dicer is a large protein, it is not suitable for production of a recombinant. On the other hand, the protein having an activity of degrading a dsRNA of the present invention is smaller than the native enzyme and compact. Thus, it is useful for production of a recombinant. In general, if an enzyme derived from a higher organism (e.g., human) is to be produced as a recombinant in cells of a bacterium (e.g., *Escherichia coli),* it may be difficult to produce the recombinant while retaining an equivalent enzymatic activity in many cases. Accordingly, the protein having an activity of degrading a dsRNA of the present invention is very useful for production in cells from an organism other than its origin.

The protein of the present invention may contain a PAZ domain. Examples thereof include, but are not limited to, a protein having amino acid 898 to amino acid 1924 in the amino acid sequence of SEQ ID NO:1 (nucleotide 2692 to nucleotide 5772 in SEQ ID NO:2), a protein (a Dicer mutant) consisting of the amino acid sequence of SEQ ID NO:17 or an amino acid sequence encoded by the nucleotide sequence of SEQ ID NO:16, and a protein (a Dicer mutant) consisting of the amino acid sequence of SEQ ID NO:18 or an amino acid sequence encoded by the nucleotide sequence of SEQ ID NO:19.

Furthermore, a more enzymatically stable one can be obtained as a mutant. Although it is not intended to limit the present invention, for example, an increased stability may be observed in case of the PAZ domain + the RNase III domain as compared with a mutant protein composed only of the RNase III domain. The activity can be retained even after more freeze-thaw cycles, and the activity can be retained in liquid state for a longer period of time using a certain storage buffer.

Although it is not intended to limit the present invention, for example, the protein of the present invention can degrade a long dsRNA to produce an siRNA that is effective for RNA interference.
The proteins having an activity of degrading a dsRNA of the present invention include one in which one or plural amino acid(s) (or nucleotide(s)) is(are) substituted, deleted, inserted or added in the amino acid sequence (or the nucleotide sequence) as long as it has the above-mentioned function.
Although it is not intended to limit the present invention, the proteins having an activity of degrading a dsRNA of the present invention include ones having the following being added to the protein: a sequence derived from an expression vector such as an expression or translation enhancing sequence (e.g., Perfect DB sequence), or an amino acid sequence such as a tag sequence for purification of an expressed protein (e.g., His tag sequence) or a sequence for removing an N-terminal additional sequence of an expressed protein (e.g., Factor Xa sequence). Examples of such proteins include, but are not limited to, a protein having an activity of degrading a dsRNA that has the amino acid sequence of SEQ ID NO: 12 or 18.
The protein having an activity of degrading a dsRNA of the present invention can convert a long dsRNA into a dsRNA of a specific length. Thus, one can prepare a dsRNA of a desired length according to the present invention by appropriately selecting the protein having an activity of degrading a dsRNA to be used. Although it is not intended to limit the present invention, the dsRNA of a specific length is exemplified by a dsRNA of a specific length from about 10 to 100 base pairs, preferably from about 15 to 30 base pairs, more preferably from about 20 to 25 base pairs.
A dsRNA used as a substrate is not completely degraded using a conventional commercially available enzyme. On the other hand, a long dsRNA as a substrate can be substantially wholly cleaved using the protein having an activity of degrading a dsRNA of the present invention. Since the dsRNA as a substrate can be wholly cleaved and the RNA interference activity of the degradation product is equivalent, scaling down of the long dsRNA as a substrate and, in its turn, omission of a step of removing undegraded dsRNA are made possible.
Furthermore, the activity of the protein having an activity of degrading a dsRNA of the present invention (for example, without limitation, a Dicer mutant) can be stably retained for a long period of time by storage in a tris-hydrochloride buffer at pH 8.5 or above in the presence of magnesium chloride either at 4°C or at -20°C.
The protein having an activity of degrading a dsRNA of the present invention may be in a form of a fusion protein with a protein having an activity of binding to a nucleic acid as described in (2) below (for example, without limitation, a protein having an activity of binding to an RNA). Although it is not intended to limit the present invention, examples thereof include a fusion protein of the above-mentioned Dicer mutant and a protein having an activity of binding to a nucleic acid (e.g., a protein having an activity of binding to an RNA).

A method for producing the protein having an activity of degrading a dsRNA of the present invention may comprise conversion of a codon into one that is suitable for expression in a host, or reinforcement for a rare codon. Although it is not intended to limit the present invention, for example, it may be a production method comprising modification of a codon in a gene to be expressed. Expression can be carried out using a gene in a state in which codons for an amino acid sequence of a protein are partially or entirely converted into ones optimal for protein expression, or using a host in a state in conformity therewith. Although it is not intended to limit the present invention, the host in a state in conformity with the state of optimal codons is exemplified by a host in which the amount of a tRNA that recognizes a specific codon is made several times larger than the amount normally produced in the cell using genetic engineering techniques. Examples of such hosts include *Escherichia coli* cells such as an *Escherichia coli* cell reinforced with tRNAs for codons for arginine (AGA, AGG), and an *Escherichia coli* cell reinforced with tRNAs for isoleucine (AUA), proline (CCC) and leucine (CUA).
A method in which expression of a protein of interest in a host is increased by modification of a codon may be used. In this case, one may take the secondary structure of an mRNA into consideration.
There is no specific limitation concerning the method as long as protein expression is increased by conversion of a codon in a gene to be expressed, reinforcement or the like according to the method.

There is no specific limitation concerning a vector used for producing the protein having an activity of degrading a dsRNA of the present invention. Any commercially available vector or expression system may be used. In particular, the pET system (Novagen) can be used, for example, although it is not intended to limit the present invention. In addition, a vector having a promoter that is capable of functioning at a low temperature can be preferably used. Examples thereof include the pCold-series vectors as described in WO 99/27117.
Any vector can be preferably used according to the production method of the present invention as long as the vector can be used to express a protein retaining a function by which a dsRNA of a specific length can be produced.
Furthermore, a vector that is capable of expressing a protein that is in a form of inclusion body upon expression of the protein but whose function can be restored by a subsequent refolding procedure may be included, provided that it is possible to obtain a protein that can finally retain a function by which a dsRNA of a specific length can be produced.
In an embodiment of the method of the present invention, for example, where the above-mentioned Dicer mutant is to be produced, the productivity and the yield of the protein in an activity-retaining form can be increased as compared with a case where the full-length conventional human Dicer is expressed.
The methods for producing the protein having an activity of degrading a dsRNA of the present invention include a method in which the protein is expressed in a form of a fusion protein with a protein having an activity of binding to a nucleic acid (for example, without limitation, a protein having an activity of binding to an RNA) as described in (2) below.

(2) Method for promoting dsRNA degradation using the protein having an activity of binding to a nucleic acid of the present invention, and method for promoting RNA synthesis using the protein
The method for promoting dsRNA degradation of the present invention in which a dsRNA of a specific length is produced is characterized in that it is carried out in the presence of a protein having an activity of binding to a nucleic acid. There is no specific limitation concerning the protein having an activity of binding to a nucleic acid as long as it results in promotion of a dsRNA degradation activity. For example, the above-mentioned protein having an activity of binding to an RNA can be preferably used.
Examples of the proteins having an activity of binding to an RNA include, but are not limited to, a cold shock protein (Csp). In particular, a cold shock protein that can function in a normal temperature range can be preferably used. A cold shock protein derived from a thermophilic bacterium or a thermostable bacterium is preferable. Although it is not intended to limit the present invention, for example, a CspB protein derived from *Thermotoga maritima* having the amino acid sequence of SEQ ID NO:9 (an amino acid sequence encoded by the nucleotide sequence of SEQ ID NO:10) can be preferably used. For example, a recombinant of the CspB protein from *Thermotoga maritima* can be produced using genetic engineering techniques according to the method described in Protein Science, 8:394-403 (1999) with *Thermotoga maritima* strain MSB8 purchased from Deutsche Sammlung von Mikroorganismen und Zellkulturen GmbH (DSM3109). A vector having a promoter capable of functioning at a low temperature can be preferably used. Examples thereof include the pCold-series vectors as described in WO 99/27117.
It is possible to promoter an activity of degrading a dsRNA by using a CspB protein and a protein having an activity of degrading a dsRNA in combination. According to the method of the present invention, it is possible to promote an activity of producing a dsRNA of a specific length for any of the proteins having an activity of degrading a dsRNA to produce a dsRNA of a specific length as described in (1) above (e.g., functional equivalents such as Dicer mutants, native Dicers or commercially available recombinant Dicers).
An activity of degrading a dsRNA can be promoted according to the method of the present invention by using a protein having an activity of binding to a nucleic acid and a protein having an activity of degrading a dsRNA in combination. The resulting degradation product has an RNA interference activity (per unit weight) equivalent to that of a degradation product obtained without using a protein having an activity of binding to a nucleic acid in combination. Thus, the use of a protein having an activity of binding to a nucleic acid is very useful for RNA interference.
A dsRNA used as a substrate is not completely degraded according to a conventional method. On the other hand, a long dsRNA as a substrate can be substantially wholly cleaved according to the method of the present invention. Since the dsRNA as a substrate can be wholly cleaved and the RNA interference activity of the degradation product is equivalent, scaling down of the long dsRNA as a substrate and, in its turn, omission of a step of removing undegraded dsRNA are made possible.
Furthermore, according to the method of the present invention, a protein having an activity of binding to a nucleic acid (e.g., a protein having an activity of synthesizing an RNA) may be in a form of a fusion protein with a protein having an activity of degrading a dsRNA.

The method for promoting RNA synthesis of the present invention is characterized in that it is carried out in the presence of a protein having an activity of binding to a nucleic acid. There is no specific limitation concerning the protein having an activity of binding to a nucleic acid as long as it results in promotion of an RNA synthesis activity of a protein having the activity. A protein having an activity of binding to a nucleic acid, a cold shock protein (Csp), can be preferably used. Although it is not intended to limit the present invention, a cold shock protein derived from a thermophilic bacterium or a thermostable bacterium can be preferably used. Examples of the cold shock proteins include, but are not limited to, a CspB protein derived from *Thermotoga maritima* having the amino acid sequence of SEQ ID NO:9 (the nucleotide sequence of SEQ ID NO:10).
For example, an RNA synthesis activity of an RNA polymerase can be promoted by including a CspB protein in an RNA synthesis system. The protein can promote the synthesis activity whether the product is a single-stranded RNA or a double-stranded RNA.

The method for promoting RNA synthesis of the present invention can be utilized for synthesis of not only a long dsRNA but also a short dsRNA (e.g., an siRNA). The method can be utilized for synthesizing, using T7 RNA polymerase or the like, an siRNA preferably of about 15 to 30 base pairs, more preferably of about 20 to 25 base pairs although it is not intended to limit the present invention.

The protein having an activity of binding to a nucleic acid promotes two types of reactions, i.e., dsRNA synthesis with a protein having an RNA synthesis activity and dsRNA degradation with a protein having a dsRNA degradation activity, according to the method of the present invention. Thus, the method can be utilized for a system for synthesizing a dsRNA which is particularly important for RNA interference or a system for producing an siRNA. In this case, the respective proteins may be individual ones or they may be in a form of a fusion protein.

(3) Composition used for the method of the present invention
The composition of the present invention is a composition for efficiently conducting a reaction of degradation into a dsRNA of a specific length and/or an RNA synthesis reaction.
The composition contains a protein having an activity of binding to a nucleic acid as described in (2) above. A cold shock protein can be preferably used as the protein having an activity of binding to a nucleic acid although it is not intended to limit the present invention. For example, a cold shock protein (Csp) derived from a thermophilic bacterium or a thermostable bacterium can be preferably used. A CspB protein derived from *Thermotoga maritima* having the amino acid sequence of SEQ ID NO:9 (an amino acid sequence encoded by the nucleotide sequence of SEQ ID NO:10) is preferable.

The composition of the present invention may contain a protein having an activity of degrading a dsRNA and/or a protein having an activity of synthesizing an RNA. One having a functional domain of a Dicer is preferable as a protein having an activity of degrading a dsRNA that is capable of producing a dsRNA of a specific length. For example, a protein consisting of RNase IIIa, RNase IIIb and dsRNA-binding domains can be preferably used. Although it is not intended to limit the present invention, for example, any of the functional equivalents such as Dicer mutants, native Dicers or commercially available recombinant Dicers as described in (1) above may be used. The composition containing a Dicer mutant may be a composition containing a protein consisting of the amino acid sequence of SEQ ID NO:4 or 17 or an amino acid sequence encoded by the nucleotide sequence of SEQ ID NO:3 or 16. It may be a composition containing a protein consisting of an amino acid sequence in which one or plural amino acid(s) is(are) substituted, deleted, inserted or added in the amino acid sequence of SEQ ID NO:4 or 17.
The protein having an activity of degrading a dsRNA may be a protein having the following being added to the protein: a sequence derived from an expression vector such as an expression or translation enhancing sequence (e.g., Perfect DB sequence), or an amino acid sequence such as a tag sequence for purification of an expressed protein (e.g., His tag sequence) or a sequence for removing an N-terminal additional sequence of an expressed protein (e.g., Factor Xa sequence). Examples of such proteins include, but are not limited to, a protein having an activity of degrading a dsRNA that has the amino acid sequence of SEQ ID NO:12 or 18. Furthermore, the composition of the present invention may contain a buffer for stabilizing a Dicer mutant as described in (1) above.

For example, T7 RNA polymerase, T3 RNA polymerase, SP6 RNA polymerase or the like can be preferably used as a protein having an activity of synthesizing an RNA.
In another embodiment of the composition of the present invention, the composition may contain a fusion protein of a protein having an activity of binding to a nucleic acid as described in (2) above (e.g., a protein having an activity of binding to an RNA) and a protein having an activity of degrading a dsRNA that is capable of producing a dsRNA of a specific length and/or a fusion protein of the protein having an activity of binding to a nucleic acid and a protein having an activity of synthesizing an RNA.
A reaction of efficient degradation into a dsRNA of a specific length and/or synthesis of a single-stranded or double-stranded RNA can be conveniently carried out using the composition of the present invention.

An embodiment of the composition of the present invention is a composition that can be utilized for synthesis of not only a long dsRNA but also a short dsRNA (e.g., an siRNA). Such a composition is effective for synthesizing a dsRNA of about 10 to 100 base pairs, preferably of about 15 to 30 base pairs, more preferably of about 20 to 25 base pairs.

(4) Kit used for the method of the present invention
The kit used for the method of the present invention is a kit for efficiently conducting a reaction of degradation into a dsRNA of a specific length and/or an RNA synthesis reaction.
The kit contains a protein having an activity of binding to a nucleic acid as described in (2) above. A cold shock protein can be preferably used as the protein having an activity of binding to a nucleic acid although it is not intended to limit the present invention. For example, a cold shock protein (Csp) derived from a thermophilic bacterium or a thermostable bacterium can be preferably used. A CspB protein derived from *Thermotoga maritima* having the amino acid sequence of SEQ ID NO:9 can be preferably used.

The kit of the present invention may contain a protein having an activity of degrading a dsRNA which as an activity of producing a dsRNA of a specific length and/or a protein having an activity of synthesizing an RNA. One as described in (3) above can be preferably used as the protein having an activity of degrading a dsRNA and/or the protein having an activity of synthesizing an RNA. Furthermore, the kit of the present invention may contain a buffer for stabilizing a Dicer mutant as described in (1) above.
In another embodiment of the kit of the present invention, the kit may contain a fusion protein of a protein having an activity of binding to a nucleic acid as described in (2) above (e.g., a protein having an activity of binding to an RNA) and a protein having an activity of degrading a dsRNA that is capable of producing a dsRNA of a specific length and/or a fusion protein of the protein having an activity of binding to a nucleic acid and a protein having an activity of synthesizing an RNA.
The kit of the present invention may contain a component other than those as described above such as a reagent for purifying a dsRNA of a specific length produced as a result of the reaction, or a reagent for transferring it into a biological sample. Although it is not intended to limit the present invention, for example, it may contain a reagent for purifying an siRNA of about 21 base pairs, a regent for transferring it into a biological sample or the like.
A reaction of efficient degradation into a dsRNA of a specific length and/or synthesis of an RNA can be conveniently carried out using the kit of the present invention.

An embodiment of the kit of the present invention is a kit that can be utilized for synthesis of not only a long dsRNA but also a short dsRNA (e.g., an siRNA). Such a kit is effective for synthesizing a dsRNA of about 10 to 100 base pairs, preferably of about 15 to 30 base pairs, more preferably of about 20 to 25 base pairs.

### Examples

The following Examples illustrate the present invention in more detail, but are not to be construed to limit the scope thereof.
Among the procedures described herein, basic procedures including preparation of plasmids and restriction enzyme digestion were carried out as described in T. Maniatis et al. (eds.), Molecular Cloning: A Laboratory Manual 3rd ed., Cold Spring Harbor Laboratory (2001).

### Example 1: Expression of RNase III domain of human Dicer

### (1) Construction of expression vector

An expression vector was constructed as follows in order to express a polypeptide consisting of amino acid 1271 to amino acid 1924 (nucleotide 3811 to nucleotide 5772) in the N-terminal portion of the amino acid sequence of human Dicer as shown in SEQ ID NO:1.
First, synthetic primers 1 and 2 (SEQ ID NOS:5 and 6) were synthesized using a DNA synthesizer based on the nucleotide sequence available to the public under GenBank accession no. AB028449, and purified according to a conventional method. The synthetic primer 1 is a synthetic DNA that has a recognition sequence for a restriction enzyme KpnI at nucleotide 9 to nucleotide 14, and a nucleotide sequence corresponding to amino acid 1271 to amino acid 1277 in the amino acid sequence of human Dicer (SEQ ID NO:1) at nucleotide 16 to nucleotide 36. The synthetic primer 2 has a recognition sequence for a restriction enzyme HindIII at nucleotide 9 to nucleotide 14 and a nucleotide sequence corresponding to amino acid 1919 to amino acid 1924 in the amino acid sequence of human Dicer (SEQ ID NO:1) at nucleotide 18 to nucleotide 36.

A PCR was conducted using the synthetic primers. The reaction conditions for the PCR were as follows.
Briefly, a reaction mixture of a total volume of 50 µl was prepared by adding 2 µl of a template DNA (human cDNA library, human pancreas, Takara Bio), 5 µl of 10 x LA PCR buffer (Takara Bio), 5 µl of dNTPs (Takara Bio), 10 pmol of the synthetic primer 1, 10 pmol of the synthetic primer 2, 0.5 U of Takara LA Taq (Takara Bio) and sterile water. The reaction mixture was placed in TaKaRa PCR Thermal Cycler SP (Takara Bio) and subjected to a reaction as follows: 30 cycles of 94°C for 1 minute, 55°C for 1 minute and 72°C for 3 minutes.

After reaction, 5 µl of the reaction mixture was subjected to electrophoresis on 1.0% agarose gel. The observed about 2-kbp DNA fragment of interest was recovered and purified from the electrophoresis gel and subjected to ethanol precipitation. After ethanol precipitation, the recovered DNA was suspended in 5 µl of sterile water, and doubly digested with restriction enzymes KpnI (Takara Bio) and HindIII (Takara Bio). The KpnI-HindIII digest was extracted and purified after electrophoresis on 1.0% agarose gel to obtain a KpnI-HindIII-digested DNA fragment.

Next, a vector pCold08NC2 was prepared according to the method described in Examples 1-6 of WO 99/27117.
The vector pCold08 was cleaved with the same restriction enzymes as those used upon preparation of the KpnI-HindIII-digested DNA fragment and the termini were dephosphorylated. The thus prepared vector and the KpnI-HindIII-digested DNA fragment were mixed together and ligated to each other using DNA ligation kit (Takara Bio). 20 µl of the ligation mixture was used to transform *Escherichia coli* JM109. Transformants were grown on LB medium (containing 50 µg/ml of ampicillin) containing agar at a concentration of 1.5%(w/v).

A plasmid having the inserted DNA fragment of interest was confirmed by sequencing. This recombinant plasmid was designated as pCold08 hDi-R. This plasmid is designated and indicated as pCold08 hDi-R and has been deposited under accession no. FERM BP-10074 at International Patent Organism Depositary, National Institute of Advanced Industrial Science and Technology, AIST Tsukuba Central 6, 1-1, Higashi 1-chome, Tsukuba-shi, Ibaraki 305-8566, Japan since August 11, 2003 (date of original deposit). pCold08 hDi-R is a plasmid containing a nucleotide sequence that encodes an amino acid sequence from amino acid 1271 to amino acid 1924 in the amino acid sequence of human Dicer (SEQ ID NO:1). The protein expressed from this plasmid has a Perfect DB sequence, a His tag sequence and a Factor Xa sequence. The amino acid sequence of the protein is shown in SEQ ID NO: 12, and the nucleotide sequence is shown in SEQ ID NO:13.

### (2) Expression and purification as well as preparation of samples under various buffer conditions

pCold08 hDi-R prepared in (1) above was used to transform *Escherichia coli* BL21. Transformants were grown on LB medium (containing 50 µg/ml of ampicillin) containing agar at a concentration of 1.5%(w/v). A grown colony was inoculated into 2.5 ml of LB liquid medium (containing 50 µg/ml of ampicillin) and cultured at 37°C overnight. A portion of the culture was inoculated into 100 ml of LB medium and cultured at 37°C until the logarithmic growth phase. After cultivation, the culture was shaken for 10 minutes in an incubator at 15°C, IPTG was added thereto at a final concentration of 1.0 mM, and the cultivation was continued at 15°C for 24 hours for expression induction. The cells were collected by centrifugation, and resuspended in 5 ml of a cell disruption solution (50 mM tris-hydrochloride buffer (pH 7.5), 100 mM sodium chloride, 0.5 mM EDTA, 1% Triton X-100, 1 mM dithiothreitol, 2 mM phenylmethylsulfonyl fluoride). The cells disrupted by sonication were subjected to centrifugation (11,000 rpm, 20 minutes) to separate a supernatant extract from a precipitate.

About 5 ml of the supernatant extract was further purified using a nickel column as follows.
10 ml of buffer A (20 mM tris-hydrochloride buffer (pH 7.5), 100 mM sodium chloride, 1 mM dithiothreitol, 0.1% Triton X-100) was added to and mixed with Ni-NTA agarose (Qiagen) corresponding to a resin volume of 1 ml. The mixture was centrifuged at 1,500 rpm for several minutes. The supernatant was discarded and about 1 ml of the resin was collected. About 5 ml of the supernatant prepared from the disrupted cells was added to the resin, and mixed gently using a rotary shaker at 4°C for about one hour. Then, the resin to which the protein of interest had been adsorbed was filled in a ϕ 15-mm column, and washed twice with 5 ml of buffer A. The resin was then washed with 5 ml of buffer B (20 mM tris-hydrochloride buffer (pH 7.5), 100 mM sodium chloride, 1 mM dithiothreitol, 0.1% Triton X-100, 40 mM imidazole). The resin was further washed with 5 ml of buffer C (20 mM tris-hydrochloride buffer (pH 7.5), 800 mM sodium chloride, 1 mM dithiothreitol, 0.1% Triton X-100, 40 mM imidazole) followed by 5 ml of buffer B to remove unnecessary proteins other than the protein of interest.

After washing, elution was carried out with 3 ml of buffer D (20 mM tris-hydrochloride buffer (pH 7.5), 100 mM sodium chloride, 1 mM dithiothreitol, 0.1% Triton X-100, 100 mM imidazole). The eluate was dialyzed against 500 ml of buffer E (50 mM tris-hydrochloride buffer (pH 8.0), 100 mM sodium chloride, 0.5 mM EDTA, 0.1% Triton X-100, 1 mM dithiothreitol) and concentrated about 10-fold using Centricon (Amicon). When a portion of the purified and concentrated sample was subjected to electrophoresis on 10% SDS-polyacrylamide gel, a band for the protein of interest was observed at a position corresponding to a molecular weight of about 76,800. When Western blotting detection with an anti-His tag antibody was carried out for the sample using Anti His HRP Conjugate (Qiagen) according to the attached protocol, the band for the protein of interest was detected upon color development. Hereinafter, the human Dicer RNase III domain protein is called hDiR.

According to the above-mentioned method, the eluate with buffer D was dialyzed against buffer E. This sample is designated as Protein Sample I. Dialyses were also carried out using buffers having the following compositions in order to determine the conditions of protein shape buffer.
1. Dialysis using buffer F (50 mM tris-hydrochloride buffer (pH 8.0), 100 mM sodium chloride, 1 mM magnesium chloride, 0.1% Triton X-100, 1 mM dithiothreitol) → Protein Sample II.
2. Dialysis using buffer G (50 mM tris-hydrochloride buffer (pH 8.5), 100 mM sodium chloride, 1 mM magnesium chloride, 0.1% Triton X-100, 1 mM dithiothreitol) → Protein Sample III.
3. Dialysis using buffer H (50 mM tris-hydrochloride buffer (pH 8.8), 100 mM sodium chloride, 1 mM magnesium chloride, 0.1% Triton X-100, 1 mM dithiothreitol) → Protein Sample IV.

### Example 2: Measurement of activity of degrading dsRNA

### (1) Preparation of reaction mixture

Dicer activities of Protein Samples I to IV prepared in Example 1-(2) were measured. The activities were measured as follows.
A dsRNA as a substrate used for the activity measurements was synthesized using TurboScript T7 Transcription kit (GTS) according to the attached protocol.
Specifically, pDON-rsGFP was constructed by inserting a gene encoding red-shift green fluorescent protein (hereinafter referred to as GFP) (SEQ ID NO:11) from a plasmid pQBI125 (Wako Pure Chemical Industries) into a plasmid pDON-AI (Takara Bio). A PCR was carried out using pDON-rsGFP as a template as well as a synthetic primer 3 (SEQ ID NO:7) which has a T7 promoter sequence and a synthetic primer 4 (SEQ ID NO:8) to obtain an amplification product. An about 700-bp dsRNA was prepared by an RNA synthesis reaction using the resulting double-stranded DNA as a template and T7 RNA polymerase.

A reaction mixture of a total volume of 10 µl was prepared by adding 1 µg of the dsRNA prepared as described above, 1 µl of hDiR prepared in (2) above, 1 µl of 10 mM ATP solution, 1 µl of 50 mM magnesium chloride solution, 2 µl of 5 x reaction buffer (a 5-fold concentrate of the buffer used for final dialysis) and nuclease-free water.

In case of a commercially available Dicer (GTS), a reaction mixture of a total volume of 10 µl was prepared by adding 2 µl of Dicer enzyme solution, 1 µg of the dsRNA as a substrate, 1 µl of 10 mM ATP solution, 0.5 µl of 50 mM magnesium chloride solution, 4 µl of the attached reaction buffer and nuclease-free water.

After the above-mentioned reaction mixtures were prepared and reacted at 37°C for 17 hours, 5 µl each of the reaction mixtures was subjected to electrophoresis on 15% polyacrylamide gel and staining with ethidium bromide to observe cleavage products. A reaction mixture for which a degradation product of about 21 nucleotides was observed in the gel stained with ethidium bromide after electrophoresis was determined to have an activity.

### (2) Activity measurement

The activities of the reaction mixtures prepared in (1) above were measured to examine the influences on the RNase III domain protein (hDiR). Furthermore, samples immediately after the purification, or stored for five days at 4°C or -20°C were used in order to examine the stabilities in the buffers. As a result, the degradation products of about 21 nucleotides (the same size as that observed using the commercially available Dicer) were observed for Protein Sample III and Protein Sample IV stored either at 4°C or at -20°C, demonstrating that the activities were stably retained.

Based on the above, it was shown that the activity of the RNase III domain protein of human Dicer (hDiR) can be stabilized by storing it in a tris-hydrochloride buffer at pH 8.5 or above in the presence of magnesium chloride.

### Example 3: Examination of factors that contribute to production and degradation of dsRNA

### (1) A protein having an activity of binding to a nucleic acid in a normal temperature range was examined in order to examine factors that contribute to production and degradation of a dsRNA.

It was difficult to obtain such a protein having an activity of binding to a nucleic acid. Then, a CspB protein from *Thermotoga maritima* having the amino acid sequence of SEQ ID NO:9 was used as a model protein. The protein was prepared according to the method as described in Protein Science, 8:394-403 (1999).

### (2) Effect of CspB from Thermotoga maritima on dsRNA degradation

An activity of degrading a dsRNA with the addition of CspB was measured as follows.
Briefly, when hDi-R was used as an enzyme, a reaction mixture of a total volume of 10 µl was prepared by adding 1 µl of hDiR (enzyme solution) prepared in Example 1-(2), 1 µl of a CspB solution prepared in (1) above, 1 µg of the dsRNA used as a substrate in Example 2-(1), 1 µl of 10 mM ATP solution, 1 µl of 50 mM magnesium chloride solution, 2 µl of 5 x reaction buffer (250 mM tris-hydrochloride buffer (pH 8.5), 500 mM sodium chloride, 0.5% Triton X-100, 5 mM DTT) and nuclease-free water. In case of a commercially available Dicer, a reaction mixture of a total volume of 10 µl was prepared by adding 1 µg of the dsRNA as a substrate and nuclease-free water to the composition as described in the attached instructions. One from GTS, Stratagene or Invitrogen was used as a commercially available Dicer.

The CspB protein was added at a final concentration of 4.6 ng/µl, 9.2 ng/µl, 18.4 ng/µl or 92 ng/µl. 1 µl of 10 mM potassium phosphate buffer (pH 7.5) as a shape buffer for CspB was added to a control (no addition).

After the above-mentioned reaction mixtures were prepared and reacted at 37°C for 18 hours, 5 µl each of the reaction mixtures was subjected to electrophoresis on 15% polyacrylamide gel and staining with ethidium bromide to observe degradation products. Furthermore, the gel was subjected to image analysis using Total Lab ver. 1.11 (Nonlinear Dynamics) to quantify dsRNA degradation products of about 21 nucleotides. As a result, increase in the amount of degraded dsRNA due to the addition of CspB was observed using each amount of the commercially available Dicer or the RNase III domain protein (hDiR). In particular, increased degradation was observed around the concentration of 9.2 ng/µl.

Thus, it was shown that a more amount of a dsRNA degradation product can be obtained by adding CspB to a reaction mixture using any of the commercially available Dicers and the RNase III domain protein.

### (3) Effect of CspB from Thermotoga maritima on RNA synthesis

In RNA interference, it is also important to promote an activity of an RNA synthesis system for synthesis of a dsRNA. From this point of view, the effect of CspB from *Thermotoga maritima* on RNA synthesis was examined. A T7 RNA polymerase system was chosen as a model system. Influence on an RNA synthesis system was examined as follows. Briefly, an amount of transcript obtained using T7 RNA polymerase with the addition of CspB was used as an index. A plasmid was constructed by incorporating the rsGFP gene having the nucleotide sequence of SEQ ID NO:11 into pET16b (Novagen) having the T7 promoter which had been prepared according to a conventional method. A reaction mixture of a total volume of 20 µl was prepared by adding 1 µg of the constructed plasmid as a template DNA, 2 µl of 10 x T7 RNA polymerase buffer (Takara Bio), 2 µl of 50 mM DTT, 0.4 µl of RNase inhibitor (Takara Bio), 2 µl of 25 mM NTPs, 1 µl of T7 RNA polymerase (Takara Bio), 1 µl of the CspB solution and nuclease-free water. The reaction mixture was reacted at 37°C for 4 hours. The CspB protein was added at a final concentration of 90 ng/µl, 180 ng/µl, 460 ng/µl or 920 ng/µl in this Example. 1 µl of 10 mM potassium phosphate buffer (pH 7.5) as a shape buffer for CspB was added to a control (no addition).
2 µl of 10 x MOPS buffer (200 mM MOPS, 50 mM sodium acetate, 10 mM EDTA, 10 mM EGTA), 2 µl of formaldehyde, 9 µl of deionized formamide and 3 µl of nuclease-free water were added to 2 µl of the reacted sample. The mixture was incubated at 70°C for 10 minutes and then on ice for 1 minute. 2 µl of 10 x loading buffer was added thereto, and the resulting mixture was subjected to electrophoretic analysis using 1.25% agarose gel containing ethidium bromide in 1 x MOPS buffer. The gel was subjected to image analysis using Total Lab ver. 1.11 (Nonlinear Dynamics) to quantify an mRNA synthesized using T7 RNA polymerase. As a result, increase in the amount of transcript was observed for each concentration. In particular, it was confirmed that the amount of transcript in case of addition of CspB at 460 ng/µl or more was about 2-fold or more as compared with that in case of no addition, and that the amount in case of addition at 920 ng/µl was about 3-fold larger.

### (4) Effect of CspB from Thermotoga maritima on dsRNA synthesis

The influence of CspB from *Thermotoga maritima* on dsRNA synthesis was examined. The template for dsRNA synthesis prepared in Example 2-(1) was utilized in this Example. A commercially available product TurboScript T7 Transcription kit (Gene Therapy Systems) was used for transcription into RNA according to the attached protocol. The CspB protein was added at a final concentration of 90 ng/µl, 180 ng/µl, 460 ng/µl or 920 ng/µl. 1 µl of 10 mM potassium phosphate buffer (pH 7.5) as a shape buffer for CspB was added to a control (no addition). After reaction at 37 °C for 4 hours, 1 µl of DNase I was added thereto, and the reaction was continued at 37°C for 15 minutes. 1 µl of a 40-fold dilution of the reaction mixture was subjected to electrophoresis on 1% agarose gel containing ethidium bromide. The gel was subjected to quantification of the dsRNA synthesized with T7 RNA polymerase according to the method as described in (3) above. As a result, increase in the amount of transcript was observed for each concentration. In particular, it was confirmed that the amount of transcript in case of addition of CspB at 920 ng/µl or more was about 2-fold or more as compared with that in case of no addition.

Another embodiment of T7 RNA polymerase transcription system was also examined in a manner similar to that in the above-mentioned Example. Specifically, a reaction mixture of a total volume of 10 µl was prepared by adding 1 µg of the template for dsRNA synthesis prepared in Example 2-(1), 1 µl of 10 x T7 RNA polymerase buffer (Takara Bio), 1 µl of 50 mM DTT, 0.2 µl of RNase inhibitor (Takara Bio), 1 µl of 25 mM NTPs, 0.5 µl of T7 RNA polymerase (Takara Bio), 1 µl of the CspB solution and nuclease-free water. The reaction mixture was reacted at 37°C for 4 hours. The CspB protein was added at a final concentration of 90 ng/µl, 180 ng/µl, 460 ng/µl or 920 ng/µl. 1 µl of 10 mM potassium phosphate buffer (pH 7.5) as a shape buffer for CspB was added to a control (no addition). After reaction, 0.5 µl of DNase I (Takara Bio) was added to the sample, and the reaction was continued at 37°C for 15 minutes. 1 µl of a 40-fold dilution of the reaction mixture was subjected to electrophoresis on 1% agarose gel containing ethidium bromide. The gel was also subjected to quantification of the dsRNA. As a result, increase in the amount of transcript was observed for each concentration. In particular, it was confirmed that the amount of transcript in case of addition of CspB at 920 ng/µl or more was about 3-fold or more as compared with that in case of no addition.

As described above, it was shown that a more amount of transcript with T7 RNA polymerase can be obtained by adding CspB into a reaction mixture. This effect was observed upon synthesis of both a single-stranded RNA and a double-stranded RNA.

### Example 4: Expression of PAZ + RNase III domain of human Dicer

### (1) Construction of expression vector

An expression vector was constructed as follows in order to express a polypeptide consisting of amino acid 679 to amino acid 1924 (nucleotide 2035 to nucleotide 5772) in the N-terminal portion of the amino acid sequence of human Dicer as shown in SEQ ID NO:1.
First, synthetic primers 5 and 6 (SEQ ID NOS:14 and 15) were synthesized using a DNA synthesizer based on the nucleotide sequence available to the public under GenBank accession no. AB028449, and purified according to a conventional method. The synthetic primer 5 is a synthetic DNA that has a recognition sequence for a restriction enzyme KpnI at nucleotide 9 to nucleotide 14, and a nucleotide sequence corresponding to amino acid 679 to amino acid 685 in the amino acid sequence of human Dicer (SEQ ID NO:1) at nucleotide 16 to nucleotide 36. The synthetic primer 6 has a recognition sequence for a restriction enzyme HindIII at nucleotide 9 to nucleotide 14 and a nucleotide sequence corresponding to amino acid 1919 to amino acid 1924 in the amino acid sequence of human Dicer (SEQ ID NO:1) at nucleotide 18 to nucleotide 36.

A PCR was conducted using the synthetic primers. The reaction conditions for the PCR were as follows.
Briefly, a reaction mixture of a total volume of 50 µl was prepared by adding 2 µl of a template DNA (human cDNA library, human pancreas, Takara Bio), 5 µl of 10 x LA PCR buffer (Takara Bio), 5 µl of dNTPs (Takara Bio), 10 pmol of the synthetic primer 5, 10 pmol of the synthetic primer 6, 0.5 U of Takara LA Taq (Takara Bio) and sterile water. The reaction mixture was placed in TaKaRa PCR Thermal Cycler SP (Takara Bio) and subjected to a reaction as follows: 30 cycles of 94°C for 1 minute, 55°C for 1 minute and 72°C for 3 minutes.

After reaction, 5 µl of the reaction mixture was subjected to electrophoresis on 1.0% agarose gel. The observed about 2.7-kbp DNA fragment of interest was recovered and purified from the electrophoresis gel and subjected to ethanol precipitation. After ethanol precipitation, the recovered DNA was suspended in 5 µl of sterile water, and doubly digested with restriction enzymes KpnI (Takara Bio) and HindIII (Takara Bio). The KpnI-HindIII digest was extracted and purified after electrophoresis on 1.0% agarose gel to obtain a KpnI-HindIII-digested DNA fragment.

Next, the vector pCold08NC2 prepared in Example 1-(1) was cleaved with the same restriction enzymes as those used upon preparation of the KpnI-HindIII-digested DNA fragment and the termini were dephosphorylated. The thus prepared vector and the KpnI-HindIII-digested DNA fragment were mixed together and ligated to each other using DNA ligation kit (Takara Bio). 20 µl of the ligation mixture was used to transform *Escherichia coli* JM109. Transformants were grown on LB medium (containing 50 µg/ml of ampicillin) containing agar at a concentration of 1.5%(w/v).

A plasmid having the inserted DNA fragment of interest was confirmed by sequencing. This recombinant plasmid was designated as pCold08 hDi-ASI. This plasmid is designated and indicated as pCold08 hDi-ASI and has been deposited under accession number FERM BP-10076 at International Patent Organism Depositary, National Institute of Advanced Industrial Science and Technology, AIST Tsukuba Central 6, 1-1, Higashi 1-chome, Tsukuba-shi, Ibaraki 305-8566, Japan since September 26, 2003 (date of original deposit). pCold08 hDi-ASI is a plasmid containing a nucleotide sequence that encodes an amino acid sequence from amino acid 679 to amino acid 1924 in the amino acid sequence of human Dicer (SEQ ID NO:1) (the nucleotide sequence of SEQ ID NO:16, the amino acid sequence of SEQ ID NO:17). The protein expressed from this plasmid has a Perfect DB sequence, a His tag sequence and a Factor Xa sequence. The amino acid sequence of the protein is shown in SEQ ID NO:18, and the nucleotide sequence is shown in SEQ ID NO:19.

### (2) Expression and purification

pCold08 hDi-ASI prepared in (1) above was used to transform *Escherichia coli* BL21-CodonPlus-RIL strain (Stratagene). Transformants were grown on LB medium (containing 50 µg/ml of ampicillin) containing agar at a concentration of 1.5%(w/v). A grown colony was inoculated into 2.5 ml of LB liquid medium (containing 50 µg/ml of ampicillin) and cultured at 37°C overnight. A portion of the culture was inoculated into 100 ml of LB medium and cultured at 37°C until the logarithmic growth phase. After cultivation, the culture was shaken for 10 minutes in an incubator at 15°C, IPTG was added thereto at a final concentration of 1.0 mM, and the cultivation was continued at 15°C for 24 hours for expression induction. The cells were collected by centrifugation, and resuspended in 5 ml of a cell disruption solution (50 mM tris-hydrochloride buffer (pH 8.5), 100 mM sodium chloride, 1 mM magnesium chloride, 0.1% Triton X-100, 1 mM dithiothreitol, 1 mM phenylmethylsulfonyl fluoride). The cells disrupted by sonication were subjected to centrifugation (11,000 rpm, 20 minutes) to separate a supernatant extract from a precipitate.

About 5 ml of the supernatant extract was further purified using a nickel column as follows.
10 ml of buffer A (20 mM tris-hydrochloride buffer (pH 8.5), 100 mM sodium chloride, 1 mM dithiothreitol, 1 mM magnesium chloride, 0.1% Triton X-100) was added to and mixed with Ni-NTA agarose (Qiagen) corresponding to a resin volume of 1 ml. The mixture was centrifuged at 1,500 rpm for several minutes. The supernatant was discarded and about 1 ml of the resin was collected. About 5 ml of the supernatant prepared from the disrupted cells was added to the resin, and mixed gently using a rotary shaker at 4°C for about one hour. Then, the resin to which the protein of interest had been adsorbed was filled in a ϕ 15-mm column, and washed twice with 5 ml of buffer A. The resin was then washed with 5 ml of buffer B (20 mM tris-hydrochloride buffer (pH 8.5), 100 mM sodium chloride, 1 mM magnesium chloride, 1 mM dithiothreitol, 0.1% Triton X-100, 40 mM imidazole). The resin was further washed with 5 ml of buffer C (20 mM tris-hydrochloride buffer (pH 8.5), 800 mM sodium chloride, 1 mM magnesium chloride, 1 mM dithiothreitol, 0.1% Triton X-100, 40 mM imidazole) followed by 5 ml of buffer B to remove unnecessary proteins other than the protein of interest.

After washing, elution was carried out with 3 ml of buffer D (20 mM tris-hydrochloride buffer (pH 8.5), 100 mM sodium chloride, 1 mM magnesium chloride, 1 mM dithiothreitol, 0.1% Triton X-100, 100 mM imidazole). The eluate was dialyzed against 500 ml of buffer E (50 mM tris-hydrochloride buffer (pH 8.5), 100 mM sodium chloride, 1 mM magnesium chloride, 0.1% Triton X-100, 1 mM dithiothreitol) and concentrated about 10-fold using Centricon (Amicon). When a portion of the concentrate was subjected to electrophoresis on 10% SDS-polyacrylamide gel, a band for the protein of interest was observed at a position corresponding to a molecular weight of about 144,000 for a sample obtained using *Escherichia coli* BL21-CodonPlus-RIL strain (Stratagene) as a host. This sample was used for subsequent confirmation of the activity. Hereinafter, the human Dicer PAZ + RNase III domain protein is called hDi-ASI.

### (3) Measurement of activity of degrading dsRNA

The activities of degrading a dsRNA of the protein samples prepared in Example 4-(2) above were measured according to the method as described in Example 2.
As a result, a degradation product of about 21 nucleotides was observed in the gel stained with ethidium bromide after electrophoresis. Thus, activities of degrading a dsRNA were observed for the RNase III domain protein (hDiR) and the protein containing the PAZ region (hDi-ASI).

Furthermore, the protein sample was subjected to 6 or 10 cycles of freezing at -80°C and thawing at room temperature in order to examine the freeze-thaw stability. As a control, hDiR prepared in Example 1-(2) was examined in a similar manner.
As a result, the PAZ + RNase III domain protein (hDi-ASI) retained the degradation activity after 6 or 10 freeze-thaw cycles. On the other hand, the activity of hDiR was observed after up to 6 cycles of freeze-thaw cycles. Based on these results, it was confirmed that the protein acquired a more freeze-thaw stability by including the PAZ domain in addition to the RNase III domain.

### (4) Examination of factors that contribute to degradation

Influence of a protein having an activity of binding to a nucleic acid in a normal temperature range was examined for hDi-ASI prepared in Example 4-(2).
The CspB protein from *Thermotoga maritima* prepared in Example 3 was used as such a protein having an activity of binding to a nucleic acid. The effect on dsRNA degradation was determined as follows.
Briefly, a reaction mixture of a total volume of 10 µl was prepared by adding 1 µl of hDi-ASI (enzyme solution) prepared in Example 4-(2), 1 µl of the CspB solution prepared in Example 3, 1 µg of a dsRNA as a substrate, 1 µl of 10 mM ATP solution, 1 µl of 50 mM magnesium chloride solution, 2 µl of 5 x reaction buffer (250 mM tris-hydrochloride (pH 8.5), 750 mM sodium chloride, 0.5% Triton X-100, 5 mM DTT) and nuclease-free water. The CspB protein was added at a final concentration of 9.2 ng/µl, 18.4 ng/µl or 92 ng/µl. 1 µl of 10 mM potassium phosphate buffer (pH 7.5) as a shape buffer for CspB was added to a control (no addition).
After the above-mentioned reaction mixtures were prepared and reacted at 37°C for 17 hours, 5 µl each of the reaction mixtures was subjected to electrophoresis on 15% polyacrylamide gel and staining with ethidium bromide to observe cleavage products. Furthermore, the gel was subjected to image analysis using Total Lab ver. 1.11 (Nonlinear Dynamics) to quantify dsRNA degradation products of about 21 nucleotides.
As a result, increase in the amount of degraded dsRNA due to the addition of CspB was observed also in case of hDi-ASI. In particular, increased degradation was observed around the concentration of 9.2 ng/µl.

Thus, it was confirmed that CspB promoted dsRNA degradation activities of human Dicers (including native forms and mutant forms) containing the RNase III domain.

### Example 5

The RNA interference effect of an siRNA prepared using human-derived hDiR of the present invention was examined. A commercially available Dicer (GTS) was used as a control. dsRNA degradation products were prepared basically according to the method as described in Example 2-(1). Specifically, 10 µg of a dsRNA was cleaved at 37°C for 18 hours using 10 units of hDiR as described in Example 1-(2) or the commercially available Dicer. The cleavage products were purified using RNA Purification Column 1, 2 (Gene Therapy Systems) and used for assessments in RNA interference as follows.

Briefly, an appropriate number (5 x 10⁴ cells) of 293 cells were seeded into a 24-well plate containing D-MEM medium (SIGMA) supplemented with 10% FBS and 1% penicillin/streptomycin 24 hours before transfer of an siRNA, and cultured overnight in a CO₂ incubator. The cells were cultured to about 80% confluence. 3 µl of TransIT 293 Transfection Reagent (Takara Bio) was added to 50 µl of a serum-free medium. The mixture was vigorously stirred and allowed to stand at room temperature for 5 minutes. 0.3 µg of pQBI25 (Wako Pure Chemical Industries) was added thereto, and the mixture was gently mixed and allowed to stand at room temperature for 5 minutes. 4 µl of TransIT-TKO reagent was added thereto, and the mixture was gently mixed and allowed to stand at room temperature for 5 minutes. 500 ng of the siRNA was added thereto, and the mixture was gently mixed and allowed to stand at room temperature for 5 minutes to obtain a DNA/siRNA solution. The DNA/siRNA solution was added dropwise to 250 µl of D-MEM medium supplemented with 10% FBS in each well, and the mixture was mixed gently so that the solution in the well became homogeneous. 0.3 µg of pQBI25 (Wako Pure Chemical Industries) or sterile water was added alone to controls. The cells were cultured for 24 hours in a CO₂ incubator. The cells were subjected to flow cytometry using FACS Vantage (Becton-Dickinson) to determine the inhibitory effect of the transferred DNA/siRNA solution on GFP expression as compared with the control with the vector (DNA) alone. The results are shown in Table 1.

**Table 1**

| Transferred sample | Average fluorescence intensity |
|---|---|
| Control (no addition) | 8.09 |
| Control (vector alone) | 1331.44 |
| hDiR | 14.92 |
| Commercially available Dicer | 71.57 |

A less average fluorescence value as compared with the control (vector alone) as shown in Table 1 represents more RNA interference. It was confirmed that the siRNA obtained using hDiR exhibited an RNA interference effect like the one obtained using the commercially available Dicer, and the exhibited RNA interference effect was stronger than that of the one obtained using the commercially available Dicer.
Based on the above, it was confirmed that hDiR of the present invention is useful for preparation of an siRNA for RNA interference.

### Example 6

The RNA interference effect of a varying amount of an siRNA prepared using hDiR of the present invention was examined. A commercially available Dicer (Gene Therapy Systems) was used as a control. Cleavage of a dsRNA was carried out basically according to the method as described in Example 2-(1). Specifically, 10 µg of a dsRNA was cleaved at 37°C for 18 hours using 10 µl of hDiR as described in Example 1-(2) or the commercially available Dicer. CspB used in Example 3-(2) was added at a final concentration of 9.2 ng/µl and reacted.
The cleavage products were purified using RNA Purification Column 1, 2 (Gene Therapy Systems) and used for assessments in RNA interference as follows.

Briefly, an appropriate number (1.5 x 10⁵ cells) of 293 cells were seeded into a 24-well plate containing D-MEM medium (SIGMA) supplemented with 10% FBS and 1% penicillin/streptomycin 24 hours before transfer of an siRNA, and cultured overnight in a CO₂ incubator. The cells were cultured to about 95% confluence. 1 µl of Genejuice Transfection Reagent (Takara Bio) was added to 49 µl of a serum-free medium. The mixture was vigorously stirred and allowed to stand at room temperature for 5 minutes. 0.3 µg of pQBI25 (Wako Pure Chemical Industries) was added thereto, and the mixture was gently mixed and allowed to stand at room temperature for 5 minutes.
In parallel, 3 µl of Ribojuice Transfection Reagent (Takara Bio) was added to 47 µl of a serum-free medium in another tube, and the mixture was vigorously stirred. After allowing to stand at room temperature for 5 minutes, 166.7 ng, 55.6 ng or 18.5 ng of the siRNA was added thereto, and the mixture was gently mixed and allowed to stand at room temperature for 5 minutes.
One of the two types of solutions prepared as described above was added dropwise to 250 µl of D-MEM medium supplemented with 10% FBS in each well, and the mixture was mixed gently so that the solution in the well became homogeneous. The vector (DNA) or sterile water was added alone to controls. The cells were cultured for 24 hours in a CO₂ incubator. The cells were subjected to flow cytometry using FACS Vantage (Becton-Dickinson) to determine the inhibitory effect of the transferred DNA/siRNA solution on rsGFP expression as compared with the control with the vector (DNA) alone. The results are shown in Table 2.

**Table 2**

| Transferred sample | Average fluorescence intensity (relative value) |
|---|---|
| Control (no addition) | 0 |
| Control (vector alone) | 100 |
| hDiR 166.7 ng | 18.21 |
| hDiR 55.6 ng | 18.97 |
| hDiR 18.5 ng | 32.67 |
| Commercially available Dicer 166.7 ng | 17.74 |
| Commercially available Dicer 55.6 ng | 19.80 |
| Commercially available Dicer 18.5 ng | 32.34 |

A less average fluorescence intensity value as compared with the control (vector alone) as shown in Table 2 represents more RNA interference. It was confirmed that the siRNA obtained using hDiR exhibited an RNA interference effect like the one obtained using the commercially available Dicer.
Based on the above, it was confirmed that hDiR of the present invention is useful for preparation of an siRNA for RNA interference.

Total RNAs extracted from the cell samples were subjected to real-time RT-PCR to quantify the mRNAs for rsGFP for assessment in RNA interference.

Specifically, a total RNA was extracted and purified from cells cultured for 24 hours at 37°C in a CO₂ incubator after the transfer of an siRNA using trizole (Invitrogen) according to the attached protocol. A reaction mixture of a total volume of 20 µl was prepared by adding 80 ng of the total RNA, 4 µl of 5 x M-MLV buffer (Takara Bio), 1 µl of 10 mM dNTPs (Takara Bio), 100 pmol of random hexamer, 20 U of RNase Inhibitor (Takara Bio), 100 U of M-MLV Reverse Transcriptase and sterile water. The reaction mixture was placed in TaKaRa PCR Thermal Cycler SP (Takara Bio) and subjected to a reaction at 42°C for 10 minutes and then at 95°C for 2 minutes. 20 µl of a reaction dilution solution (1 x M-MLV buffer, 0.5 mM dNTP mixture) was added to the reaction mixture. 10 µl aliquots of the resulting mixture were dispensed. 2.5 µl of 10 x R-PCR buffer (Takara Bio), 0.3 µl of 250 mM Mg²⁺, 0.75 µl of 10 mM dNTPs, 1.25 U of TaKaRa Ex Taq R-PCR (Takara Bio), 2.5 µl of a 3000-fold dilution of SYBR Green (Takara Bio) in sterile water and 1.25 µl of 100% DMSO were added to 10 µl of the reaction mixture. 5 pmol each of synthetic primers for detecting β-actin (Takara Bio), GAPDH (Takara Bio), rsGFP (rsGFP-F (SEQ ID NO:20), rsGFP-R (SEQ ID NO:21)) or Neo (Neo-F (SEQ ID NO:22), Neo-R (SEQ ID NO:23)) and sterile water to a total volume of 25 µl were further added. The reaction mixtures were placed in Smart Cycler II Unit (Takara Bio), heat-denatured at 95°C for 10 seconds, and subjected to a reaction as follows: 45 cycles of 95°C for 5 seconds and 60°C for 20 seconds. The obtained data were analyzed to quantify the mRNAs for human β-actin and GAPDH as well as rsGFP and Neo from the transferred plasmid. The results are shown in Table 3.

**Table 3**

| Transferred sample | Amount of rsGFP mRNA (relative value) |
|---|---|
| Control (no addition) | 0 |
| Control (vector alone) | 100 |
| hDiR 166.7 ng | 15.92 |
| hDiR 55.6 ng | 22.72 |
| hDiR 18.5 ng | 30.61 |
| Commercially available Dicer 166.7 ng | 14.77 |
| Commercially available Dicer 55.6 ng | 30.91 |
| Commercially available Dicer 18.5 ng | 35.84 |

A less amount of rsGFP mRNA as compared with the control (vector alone) as shown in Table 3 represents more RNA interference. It was confirmed that hDiR resulted in an RNA interference effect like the commercially available Dicer.
Based on the above, it was confirmed that hDiR of the present invention is useful for preparation of an siRNA for RNA interference.

### Example 7

The samples prepared in Example 1-(2) and Example 4-(2) were assessed using a dsRNA as a substrate for measuring their dsRNA degradation activities which was prepared from a luciferase gene. A dsRNA was synthesized using TurboScript T7 Transcription kit (GTS) according to the attached protocol in a manner similar to that in Example 2-(1).

Specifically, a PCR was carried out using a plasmid pGL3-Basic vector (Promega) as a template as well as a primer dsl-1 (SEQ ID NO:24) which has a T7 promoter sequence and a primer dsl-2 (SEQ ID NO:25) to obtain the luciferase-encoding gene inserted into the plasmid pGL3-Basic vector (Promega) as an amplification product of about 500 base pairs. An about 500-bp dsRNA was prepared by an RNA synthesis reaction using the resulting double-stranded DNA as a template and T7 RNA polymerase.

The RNA interference effects of siRNAs prepared using hDiR or hDi-ASI of the present invention were examined. A commercially available Dicer (Gene Therapy Systems) was used as a control. Cleavage of a dsRNA was carried out basically according to the method as described in Example 2-(1). Specifically, 10 µg of the dsRNA was cleaved at 37°C for 18 hours using 10 µl of hDiR as described in Example 1-(2), hDi-ASI as described in Example 4-(2) or the commercially available Dicer. CspB used in Example 3-(2) was added at a final concentration of 9.2 ng/µl and reacted.
The cleavage products were purified using RNA Purification Column 1, 2 (Gene Therapy Systems) and used for assessments in RNA interference as follows.

Briefly, an appropriate number (1.5 x 10⁵ cells) of 293 cells were seeded into a 24-well plate containing D-MEM medium (SIGMA) supplemented with 10% FBS and 1% penicillin/streptomycin 24 hours before transfer of an siRNA, and cultured overnight in a CO₂ incubator. The cells were cultured to about 95% confluence. 1 µl of Genejuice Transfection Reagent (Takara Bio) was added to 49 µl of a serum-free medium. The mixture was vigorously stirred and allowed to stand at room temperature for 5 minutes. 0.5 µg of pGL3-control and 0.1 µg pRL-TK (Promega) were added thereto, and the mixture was gently mixed and allowed to stand at room temperature for 5 minutes.
In parallel, 3 µl of Ribojuice Transfection Reagent (Takara Bio) was added to 47 µl of a serum-free medium in another tube, and the mixture was vigorously stirred. After allowing to stand at room temperature for 5 minutes, 166.7 ng, 55.6 ng or 18.5 ng of the siRNA was added thereto, and the mixture was gently mixed and allowed to stand at room temperature for 5 minutes.
One of the two types of solutions prepared as described above was added dropwise to 250 µl of D-MEM medium supplemented with 10% FBS in each well, and the mixture was mixed gently so that the solution in the well became homogeneous. The vector (DNA) or sterile water was added alone to controls. The cells were cultured for 24 hours in a CO₂ incubator. The cells were subjected to an assay using Dual Luciferase Reporter assay kit (Promega) to determine the inhibitory effect of the addition of the siRNA on GL3 protein expression as compared with the control with the vector (DNA) alone. The results are shown in Table 4.

**Table 4**

| Transferred siRNA sample | GL3 expression level (relative value) |
|---|---|
| Control (no addition) | 0 |
| Control (vector alone) | 100 |
| hDiR 166.7 ng | 25.70 |
| hDiR 55.6 ng | 38.90 |
| hDiR 18.5 ng | 74.51 |
| hDi-ASI 166.7 ng | 25.87 |
| hDi-ASI 55.6 ng | 25.00 |
| hDi-ASI 18.5 ng | 31.09 |
| Commercially available Dicer 166.7 ng | 25.11 |
| Commercially available Dicer 55.6 ng | 30.15 |
| Commercially available Dicer 18.5 ng | 55.41 |

It is considered that a less GL3 expression level as compared with the control (vector alone) as shown in Table 4 represents more RNA interference. It was confirmed that the siRNA obtained using hDiR or hDi-ASI exhibited an RNA interference effect like the one obtained using the commercially available Dicer.
Based on the above, it was confirmed that hDiR and hDi-ASI of the present invention are useful for preparation of an siRNA for RNA interference.

### Example 8

The RNA interference effects of siRNAs prepared using hDiR or hDi-ASI of the present invention with or without the addition of CspB were examined. The procedure followed the method as described in Example 7 using luciferase. A commercially available Dicer (Gene Therapy Systems) was used as a control. Cleavage of a dsRNA was carried out basically according to the method as described in Example 2-(1). Specifically, 10 µg of the dsRNA was cleaved at 37°C for 18 hours using 10 µl of hDiR prepared as described in Example 1-(2), hDi-ASI prepared as described in Example 4-(2) or the commercially available Dicer. The reactions were carried out with or without the addition of CspB used in Example 3-(2) at a final concentration of 9.2 ng/µl.
The cleavage products were purified using RNA Purification Column 1, 2 (Gene Therapy Systems) and used for assessments in RNA interference as follows.

**Table 5**

| Transferred siRNA sample | GL3 expression level (relative value) |
|---|---|
| Control (no addition) | 0 |
| Control (vector alone) | 100 |
| hDiR 55.6 ng + CspB | 9.66 |
| hDiR 55.6 ng | 16.40 |
| hDi-ASI 55.6 ng + CspB | 14.31 |
| hDi-ASI 55.6 ng | 13.19 |
| Commercially available Dicer 55.6 ng + CspB | 10.40 |
| Commercially available Dicer 55.6 ng | 12.07 |

It is considered that a less GL3 expression level as compared with the control (vector alone) as shown in Table 5 represents more RNA interference. It was shown that there was no difference in RNA interference effect between samples with or without the addition of CspB.
Based on the above, it was confirmed that CspB of the present invention does not influence the quality of an siRNA for RNA interference upon preparation of the siRNA.

### Example 9: Stability of enzyme

The PAZ domain + RNase III domain protein (hDi-ASI) purified in Example 4-(2) was stored, with the addition of CspB at a final concentration of 92 ng/µl, in a storage buffer I (50 mM tris-hydrochloride buffer (pH 8.5), 250 mM sodium chloride, 1 mM magnesium chloride, 0.1 mM DTT, 0.1% Triton X-100, 50% glycerol). Samples were taken at an interval and their dsRNA degradation activities were measure as follows. A reaction mixture of a total volume of 10 µl was prepared by adding 1 µg of the dsRNA prepared in Example 2-(1), 1 µl of a sample from the protein prepared in 4-(2) above, 2 µl of 5 x reaction buffer (100 mM tris-hydrochloride buffer (pH 8.5), 750 mM sodium chloride, 12.5 mM magnesium chloride) and nuclease-free water. After the reaction mixture was reacted at 37°C for 18 hours, 5 µl of the reaction mixture was subjected to electrophoresis on 15% polyacrylamide gel and staining with ethidium bromide to observe the cleavage product. As a result, a degradation product of about 21 base pairs was observed for a sample stored for 6 months or longer, indicating its dsRNA degradation activity. The activity of the RNase III domain protein (hDiR) stored in the same buffer with the addition of CspB was retained for 3 months or more.

### Example 10: Construction of pCold14-TmCspB, cultivation of recombinant, and purification

### (1) Construction of pColdl4-TmCspB

CspB from *Thermotoga maritima* (hereinafter referred to as TmCspB) was cloned as follows referring to the sequences as described in Protein Science, 8:394-403 (1999). The amino acid sequence and the nucleotide sequence are shown in SEQ ID NO:9 and SEQ ID NO:10, respectively.
First, synthetic primers E and F (SEQ ID NOS:26 and 27) were synthesized using a DNA synthesizer, and purified according to a conventional method. The synthetic primer E is a synthetic DNA that has a recognition sequence for a restriction enzyme NdeI at nucleotide 11 to nucleotide 16, and a nucleotide sequence corresponding to amino acid 1 to amino acid 7 in the amino acid sequence of TmCspB (SEQ ID NO:26) at nucleotide 14 to nucleotide 34. The synthetic primer F is a synthetic DNA that has a recognition sequence for a restriction enzyme BamHI at nucleotide 11 to nucleotide 16 and a nucleotide sequence corresponding to amino acid 61 to amino acid 66 in the amino acid sequence of TmCspB (SEQ ID NO:26) at nucleotide 20 to nucleotide 37.

A PCR was conducted using the synthetic primers. The reaction conditions for the PCR were as follows.
Briefly, a reaction mixture of a total volume of 50 µl was prepared by adding 1 µl (50 ng) of the template DNA as described in Example 3-(1), 5 µl of 10 x Ex Taq buffer (Takara Bio), 5 µl of dNTPs (Takara Bio), 10 pmol of the synthetic primer E, 10 pmol of the synthetic primer F, 0.5 U of Takara Ex Taq (Takara Bio) and sterile water. The reaction mixture was placed in TaKaRa PCR Thermal Cycler SP (Takara Bio) and subjected to a reaction as follows: 30 cycles of 94°C for 1 minute, 55°C for 1 minute and 72°C for 1 minute.
After reaction, 5 µl of the reaction mixture was subjected to electrophoresis on 3.0% agarose gel, and an about 220-bp DNA fragment of interest was observed. The remaining PCR reaction mixture was subjected to electrophoresis, and the fragment was recovered, purified and subjected to ethanol precipitation. After ethanol precipitation, the recovered DNA was suspended in 5 µl of sterile water, and doubly digested with restriction enzymes NdeI (Takara Bio) and BamHI (Takara Bio). The NdeI-BamHI digest was extracted and purified after electrophoresis on 3.0% agarose gel to obtain a NdeI-BamHI-digested DNA fragment.

Next, a vector pCold04NC2 was prepared according to the method described in Examples 1-6 of WO 99/27117 (hereinafter, the vector pCold04NC2 is called a vector pColdl4) .

The vector pCold14 was cleaved with the same restriction enzymes as those used upon preparation of the above-mentioned DNA fragment and the termini were dephosphorylated. The thus prepared vector and the NdeI-BamHI-digested DNA fragment were mixed together and ligated to each other using DNA ligation kit (Takara Bio). 20 µl of the ligation mixture was used to transform *Escherichia coli* JM109. Transformants were grown on LB medium (containing 50 µg/ml of ampicillin) containing agar at a concentration of 1.5%(w/v).
A plasmid having the inserted DNA fragment of interest was confirmed by sequencing.

### (2) Cultivation in 5-liter jar and purification of CspB

pColdl4-TmCspB prepared in Example 10-(1) above was used to transform *Escherichia coli* BL21. Transformants were grown on LB medium (containing 50 µg/ml of ampicillin) containing agar at a concentration of 1.5%(w/v). A grown colony was inoculated into 30 ml of LB liquid medium (0.3 g of bacto-tryptone, 0.15 g of bacto-yeast extract, 0.15 g of NaCl, 1.5 mg of ampicillin) and cultured at 37°C overnight. 30 ml of the culture was inoculated into 3 liters of LB liquid medium (30 g of bacto-tryptone, 15 g of bacto-yeast extract, 15 g of NaCl, 150 mg of ampicillin) in a 5-liter jar fermentor (Able) and cultured at 37°C at 150 rpm with aeration at 0.5 liter/minute until the logarithmic growth phase. Thereafter, the culture was cooled to 15°C. After cooling, IPTG was added thereto at a final concentration of 1.0 mM, and the cultivation was continued at 15°C at 150 rpm with aeration at 0.5 liter/minute for 24 hours for expression induction. The cells were collected by centrifugation to obtain 11 g of wet cells. The wet cells (11 g) were resuspended in 44 ml of buffer A (20 mM tris-hydrochloride buffer (pH 8.0)). The cells disrupted by sonication were subjected to centrifugation (10,000 x g, 20 minutes) to separate a supernatant extract from a precipitate. The supernatant extract was further subjected to ultracentrifugation (70,000 x g, 20 minutes) to separate a supernatant extract from a precipitate.

About 53 ml of the supernatant extract was heated in a water bath (70°C, 10 minutes), and centrifuged (10,000 x g, 20 minutes) to separate a supernatant from a precipitate. NaCl at a final concentration of 200 mM was added to the heated supernatant. 10 ml of AnionExchanger DE52 equilibrated with buffer B (20 mM tris-hydrochloride buffer (pH 8.0), 200 mM sodium chloride) was further added thereto. The resulting mixture was gently stirred at 4°C overnight. The mixture was centrifuged (3,000 x g, 20 minutes) to separate a supernatant from a precipitate. The supernatant was filtrated through a glass filter. 45 ml of the filtrate was dialyzed against 3 liters of buffer A (20 mM tris-hydrochloride buffer (pH 8.0)).

50 ml of the dialysate was added to Q-Sepharose F.F. (Amersham Biosciences) corresponding to a resin volume of 100 ml filled in a ϕ 20-mm column. The column was washed with 400 ml of buffer A (20 mM tris-hydrochloride buffer (pH 8.0)). A fraction not adsorbed to the column was collected and passed through UF 30,000-cut Centriprep YM-30 (Millipore). 260 ml of the UF-passed solution was added to 40 ml of Heparin Sepharose CL-6B (Amersham Biosciences) filled in a ϕ 30-mm column. The column was washed with 160 ml of buffer A (20 mM tris-hydrochloride buffer (pH 8.0)). The protein of interest was eluted with a gradient of 0 to 1 M sodium chloride in 200 ml of buffer A (20 mM tris-hydrochloride buffer (pH 8.0)). A fraction with which the protein of interest having a molecular weight of about 7,500 was observed upon tricine-SDS-polyacrylamide gel electrophoresis was collected, and dialyzed against 3 liters of buffer A (20 mM tris-hydrochloride buffer (pH 8.0)). 65 ml of the dialysate was concentrated about 144-fold using UF 3,000-cut Centriprep YM-3 (Millipore) to obtain an about 450 µl of a protein sample. An equal amount of glycerol was added to the protein sample to obtain 850 µl of a 50%(w/w) glycerol solution. A portion of the solution was subjected to tricine-SDS-polyacrylamide gel electrophoresis, and a single stained band for the protein of interest was observed at a position corresponding to a molecular weight of about 7, 500.

### (3) Increase in cleavage activity by CspB protein

Influence of a protein having an activity of binding to a nucleic acid (CspB) was examined using the protein samples prepared in Example 1-(2) and Example 4-(2). In this case, TmCspB prepared in Example 10-(2) was used.
Specifically, a reaction mixture of a total volume of 10 µl was prepared by adding 1 µl of the protein sample prepared in Example 1-(2) or Example 4-(2) (hDi-R enzyme solution or hDi-ASI enzyme solution), 1 µl of the CspB solution (92 ng/µl) prepared in Example 10-(2), 1 µg of the dsRNA as a substrate prepared in Example 2-(1), 2 µl of 5 x reaction buffer (100 mM tris-hydrochloride buffer (pH 8.5), 750 mM sodium chloride, 12.5 mM magnesium chloride) and nuclease-free water. The final concentration of the CspB protein was 9.2 ng/µl. A similar procedure was carried out using a commercially available Dicer as a control. In case of the commercially available Dicer (GTS), a reaction mixture of a total volume of 10 µl was prepared by adding 2 µl of an enzyme solution, 1 µl of the CspB solution, 1 µg of the dsRNA as a substrate, 1 µl of 10 mM ATP solution, 0.5 µl of 50 mM magnesium chloride solution, 4 µl of the attached reaction buffer and nuclease-free water. 1 µl of nuclease-free water was added in place of TmCspB for a negative control.
After the above-mentioned reaction mixtures were prepared and reacted at 37°C for 18 hours, 5 µl each of the reaction mixtures was subjected to electrophoresis on 15% polyacrylamide gel and staining with ethidium bromide to observe cleavage products. The gel was subjected to image analysis using Total Lab ver. 1.11 (Nonlinear Dynamics) to quantify dsRNA degradation products of about 21 nucleotides.
As a result, increase in the amount of degraded dsRNA observed using TmCspB expressed and purified in Example 3-(1) was also observed using TmCspB expressed and purified in Example 10-(2).

### Example 11: Cultivation in 30-liter jar and purification of hDi-ASI

### (1) Expression and purification

pCold08 hDi-ASI prepared in Example 4-(1) was used to transform *Escherichia coli* BL21. Transformants were grown on LB medium (containing 50 µg/ml of ampicillin) containing agar at a concentration of 1.5%(w/v). A grown colony was inoculated into 200 ml of TB liquid medium (2.4 g of bacto-tryptone, 4.8 g of bacto-yeast extract, 0.8 ml of glycerol, 17 mM KH₂PO₄, 72 mM K₂HPO₄, 10 mg of ampicillin) and cultured at 37°C overnight. 200 ml of the culture was inoculated into 20 liters of TB liquid medium (240 g of bacto-tryptone, 480 g of bacto-yeast extract, 80 ml of glycerol, 17 mM KH₂PO₄, 72 mM K₂HPO₄, 1 g of ampicillin) in a 30-liter jar fermentor (B. E. Marubishi) and cultured at 37°C at 100 rpm with aeration at 6 liter/minute until the logarithmic growth phase. Thereafter, the culture was cooled to 15°C. After cooling, IPTG was added thereto at a final concentration of 1.0 mM, and the cultivation was continued at 15°C at 100 rpm with aeration at 6 liter/minute for 24 hours for expression induction. The cells were collected by centrifugation to obtain 26 g of wet cells. A portion of the wet cells (12 g) were resuspended in 48 ml of cell disruption solution (50 mM tris-hydrochloride buffer (pH 8.5), 100 mM sodium chloride, 1 mM magnesium chloride, protease inhibitor (Complete, EDTA-free, Boehringer Mannheim)). The cells disrupted by sonication were subjected to centrifugation (12,000 rpm, 30 minutes) to separate a supernatant extract from a precipitate.
About 56 ml of the supernatant extract was further purified using a nickel column as follows.

50 ml of the cell disruption solution was added to and mixed with Ni-NTA agarose (Qiagen) corresponding to a resin volume of 16 ml. The mixture was centrifuged at 1,500 rpm for several minutes. The supernatant was discarded. This procedure was repeated twice, and about 8 ml of the resin was collected. About 56 ml of the supernatant prepared from the disrupted cells was added to the resin, and mixed gently using a rotary shaker at 4°C for about one hour. Then, the resin to which the protein of interest had been adsorbed was filled in a ϕ 20-mm column, and washed with 40 ml of a cell disruption solution (50 mM tris-hydrochloride buffer (pH 8.5), 100 mM sodium chloride, 1 mM magnesium chloride, protease inhibitor (Complete, EDTA-free, Boehringer Mannheim)). The resin was then washed with 40 ml of buffer A (20 mM tris-hydrochloride buffer (pH 8.5), 100 mM sodium chloride, 1 mM magnesium chloride, 10% glycerol, 20 mM imidazole), 40 ml of buffer B (20 mM tris-hydrochloride buffer (pH 8.5), 800 mM sodium chloride, 1 mM magnesium chloride, 10% glycerol, 20 mM imidazole), and 40 ml of buffer A to remove unnecessary proteins other than the protein of interest.
After washing, elution was carried out with 24 ml of buffer C (20 mM tris-hydrochloride buffer (pH 8.5), 100 mM sodium chloride, 1 mM magnesium chloride, 10% glycerol, 100 mM imidazole). The eluate was dialyzed against 300 ml of buffer D (20 mM tris-hydrochloride buffer (pH 8.5), 100 mM sodium chloride, 1 mM magnesium chloride, 10% glycerol).

The dialyzed enzyme solution was added to 1 ml of Heparin Sepharose CL-6B (Amersham Biosciences) filled in a ϕ 10-mm column. The column was washed with 5 ml of buffer D (20 mM tris-hydrochloride buffer (pH 8.5), 100 mM sodium chloride, 1 mM magnesium chloride, 10% glycerol). Elution of protein was carried out using 5 ml of buffer E (20 mM tris-hydrochloride buffer (pH 8.5), 200 mM sodium chloride, 1 mM magnesium chloride, 10% glycerol), 5 ml of buffer F (20 mM tris-hydrochloride buffer (pH 8.5), 400 mM sodium chloride, 1 mM magnesium chloride, 10% glycerol) and 5 ml of buffer G (20 mM tris-hydrochloride buffer (pH 8.5), 800 mM sodium chloride, 1 mM magnesium chloride, 10% glycerol). Each of the eluted samples was concentrated about 20-fold using Centricon YM-10 (Amicon) to obtain an about 250 µl of a protein sample. When a portion of the protein sample was subjected to electrophoresis on 10% SDS-polyacrylamide gel, a band for the protein of interest was observed at a position corresponding to a molecular weight of about 144,000. This sample was used for subsequent confirmation of the activity. Comparison of the activity corresponding to 100 ml of the cultures for the sample expressed and purified as described above with that expressed and purified using *Escherichia coli* BL21-CodonPlus-RIL strain (Stratagene) as a host in Example 4-(2) indicated an about 2-fold greater yield.

### (2) Measurement of dsRNA degradation activity

Using the protein sample prepared in Example 11-(1) above, a reaction mixture of a total volume of 10 µl was prepared by adding 1 µg of the dsRNA prepared in Example 2-(1), 1 ul of the protein sample prepared in 11-(1), 2 µl of 5 x reaction buffer (100 mM tris-hydrochloride buffer (pH 8.5), 750 mM sodium chloride, 12.5 mM magnesium chloride) and nuclease-free water. After the reaction mixture was reacted at 37°C for 18 hours, 5 µl of the reaction mixture was subjected to electrophoresis on 15% polyacrylamide gel and staining with ethidium bromide to observe the cleavage product. As a result, a degradation product of about 21 base pairs was observed, indicating its dsRNA degradation activity.

### (3) Increase in cleavage activity by CspB protein

Influence of a protein having an activity of binding to a nucleic acid (CspB) was examined using the protein sample prepared in Example 11-(1).
Specifically, like the procedure in Example 10-(3), a reaction mixture of a total volume of 10 µl was prepared by adding 1 µl of the protein sample prepared in Example 11-(1) (enzyme solution), 1 µl of the CspB solution prepared in Example 10-(2), 1 µg of the dsRNA as a substrate prepared in Example 2-(1), 2 µl of 5 x reaction buffer (100 mM tris-hydrochloride buffer (pH 8.5), 750 mM sodium chloride, 12.5 mM magnesium chloride) and nuclease-free water. The final concentration of the CspB protein was 9.2 ng/µl. A similar procedure was carried out using a commercially available Dicer as a control. In case of the commercially available Dicer (GTS), a reaction mixture of a total volume of 10 µl was prepared by adding 2 µl of an enzyme solution, 1 µl of the CspB solution, 1 µg of the dsRNA as a substrate, 1 µl of 10 mM ATP solution, 0.5 µl of 50 mM magnesium chloride solution, 4 µl of the attached reaction buffer and nuclease-free water. Nuclease-free water was added in place of CspB for a control.

After the above-mentioned reaction mixtures were prepared and reacted at 37°C for 18 hours, 5 µl of the reaction mixture was subjected to electrophoresis on 15% polyacrylamide gel and staining with ethidium bromide to observe cleavage products. The gel was subjected to image analysis using Total Lab ver. 1.11 (Nonlinear Dynamics) to analyze a dsRNA degradation product of about 21 nucleotides and an uncleaved dsRNA.
As a result, when the mutant protein containing the PAZ + RNase III domain of the present invention (hDi-ASI) was used, increase in the amount of degraded dsRNA due to the addition of CspB was observed, and almost no uncleaved substrate existed, indicating almost complete degradation. On the other hand, in case of the commercially available Dicer, increase in the amount of degraded dsRNA was observed although about half of the substrate remained uncleaved.
Thus, it was shown that the substrate could be completely degraded by adding CspB when the protein of the present invention was used.

### Example 12: RNAi effect of cleavage product obtained using hDi-ASI

The RNA interference effect of an siRNA prepared using the protein in Example 11-(1) was examined. A commercially available Dicer (GTS) was used as a control. dsRNA degradation products were prepared basically according to the method as described in Example 11-(3). In case of hDi-ASI, CspB prepared in Example 10-(2) was added at a final concentration of 9.2 ng/µl. Specifically, 10 µg of the dsRNA prepared in Example 2-(1) was cleaved at 37°C for 18 hours using the enzyme as described in Example 11-(1) with the addition of CspB or the commercially available Dicer. The cleavage products were purified using Microcon-100 and Micropure-EZ (both from Takara Bio), and used for assessments in RNA interference as follows.

Briefly, an appropriate number (1.5 x 10⁵ cells) of 293 cells were seeded into a 24-well plate containing D-MEM medium (SIGMA) supplemented with 10% FBS and 1% penicillin/streptomycin 24 hours before transfer of an siRNA, and cultured overnight in a CO₂ incubator. The cells were cultured to about 95% confluence. 1 µl of Genejuice Transfection Reagent (Takara Bio) was added to 49 µl of a serum-free medium. The mixture was vigorously stirred and allowed to stand at room temperature for 5 minutes. 0.3 µg of pQBI25 (Takara Bio) was added thereto, and the mixture was gently mixed and allowed to stand at room temperature for 5 minutes.
In parallel, 3 µl of Ribojuice Transfection Reagent (Takara Bio) was added to 47 µl of a serum-free medium in another tube, and the mixture was vigorously stirred. After allowing to stand at room temperature for 5 minutes, 55.6 ng of the siRNA was added thereto, and the mixture was gently mixed and allowed to stand at room temperature for 5 minutes.

One of the two types of solutions prepared as described above was added dropwise to 250 µl of D-MEM medium supplemented with 10% FBS in each well, and the mixture was mixed gently so that the solution in the well became homogeneous. The vector (DNA) or sterile water was added alone to controls. The cells were cultured for 24 hours in a CO₂ incubator. The cells were subjected to flow cytometry using FACS Vantage (Becton-Dickinson) to determine the inhibitory effect of the transferred DNA/siRNA solution on rsGFP expression as compared with the control with the vector (DNA) alone. The results are shown in Table 6.

**Table 6**

| Transferred sample | Average fluorescence intensity |
|---|---|
| Control (no addition) | 3.52 |
| Control (vector alone) | 1120.08 |
| hDi-ASI + CspB | 93.09 |
| Commercially available Dicer | 278.59 |

A less average fluorescence value as compared with the control (vector alone) as shown in Table 6 represents more RNA interference. It was shown that the siRNA obtained using hDiR-ASI + CspB exhibited an RNAi effect like the one obtained using the commercially available Dicer and, therefore, it was effective for RNAi. Furthermore, it was confirmed that an siRNA could be efficiently produced, and a greater RNAi effect could be achieved as compared with a commercially available enzyme provided that the same amount of an siRNA was used.
Based on the above, it was confirmed that the protein prepared according to the method of the present invention is useful for preparation of an siRNA for RNA interference.

### Industrial Applicability

The present invention provides a protein having an activity of degrading a dsRNA to produce a dsRNA of a specific length. Furthermore, a method for promoting degradation into a dsRNA of a specific length and/or a method for promoting synthesis of an RNA which is useful for RNA interference or the like is provided according to the method of the present invention. In addition, a composition and a kit which can be used to conveniently carry out the method for promoting degradation into a dsRNA of a specific length and/or the method for promoting synthesis of an RNA of the present invention are provided.

### Sequence Listing Free Text

SEQ ID NO:3; A gene encoding human dicer mutant
SEQ ID NO:4; An amino acid sequence of human dicer mutant
SEQ ID NO:5; Synthetic primer 1 to amplify a gene encoding human dicer mutant
SEQ ID NO:6; Synthetic primer 2 to amplify a gene encoding human dicer mutant
SEQ ID NO:7; Synthetic primer 3 to amplify a gene encoding red-shifted green fluorescence protein
SEQ ID NO:8; Synthetic primer 4 to amplify a gene encoding red-shifted green fluorescence protein
SEQ ID NO:14; Synthetic primer 5 to amplify a gene encoding human dicer mutant
SEQ ID NO:15; Synthetic primer 6 to amplify a gene encoding human dicer mutant
SEQ ID NO:16; A gene encoding human dicer mutant
SEQ ID NO:17; An amino acid sequence of human dicer mutant
SEQ ID NO:18; An amino acid sequence of human dicer mutant
SEQ ID NO:19; A gene encoding human dicer mutant
SEQ ID NO:20; Synthetic primer rsGFP-F to amplify a gene encoding rsGFP
SEQ ID NO:21; Synthetic primer rsGFP-R to amplify a gene encoding rsGFP
SEQ ID NO:22; Synthetic primer Neo-F to amplify a gene encoding Neo
SEQ ID NO:23; Synthetic primer Neo-R to amplify a gene encoding Neo
SEQ ID NO:24; Synthetic primer dsl-1 to amplify a gene encoding luciferase
SEQ ID NO:25; Synthetic primer dsl-2 to amplify a gene encoding luciferase
SEQ ID NO:26; Synthetic primer E to amplify a gene encoding CspB
SEQ ID NO:27; Synthetic primer F to amplify a gene encoding CspB

## Claims

1. A protein having an activity of degrading a dsRNA, which has an activity of acting on a dsRNA to produce a dsRNA of a specific length.

2. The protein according to claim 1, which has a functional domain of a Dicer.

3. The protein according to claim 2, wherein the functional domain of a Dicer consists of RNase IIIa, RNase IIIb and dsRNA-binding domains.

4. The protein according to claim 3, which further contains a PAZ domain.

5. The protein according to claim 1, wherein the dsRNA of specific length is a dsRNA of about 15 to 30 base pairs.

6. The protein according to claim 1, which is a protein consisting of the amino acid sequence of SEQ ID NO:4 or 17, or an amino acid sequence encoded by the nucleotide sequence of SEQ ID NO:3 or 16.

7. The protein according to claim 1, which is a protein consisting of an amino acid sequence in which one or plural amino acid(s) is(are) substituted, deleted, inserted or added in the amino acid sequence of SEQ ID NO:4 or 17.

8. A method for producing the protein having an activity of degrading a dsRNA defined by claim 1, the method comprising converting a codon into one that is suitable for expression in a host, or reinforcing a host to be used for a rare codon.

9. A method for producing the protein having an activity of degrading a dsRNA defined by claim 1, the method comprising expressing the protein using a cold-inducible vector.

10. A kit containing the protein having an activity of degrading a dsRNA defined by claim 1.

11. A method for degrading a dsRNA, the method comprising allowing a protein having an activity of degrading a dsRNA to act on a dsRNA in the presence of a protein having an activity of binding to a nucleic acid to produce a dsRNA of a specific length.

12. The method according to claim 11, wherein the protein having an activity of binding to a nucleic acid and the protein having an activity of degrading a dsRNA are a fusion protein.

13. The method according to claim 11, wherein the protein having an activity of binding to a nucleic acid is a protein having an activity of binding to an RNA.

14. The method according to claim 13, wherein the protein having an activity of binding to an RNA is a cold shock protein.

15. The method according to claim 14, wherein the cold shock protein is derived from a thermophilic bacterium or a thermostable bacterium.

16. The method according to claim 15, wherein the cold shock protein is cold shock protein B from *Thermotoga maritima.*

17. The method according to claim 11, wherein the dsRNA of specific length is a dsRNA of about 15 to 30 base pairs.

18. The method according to claim 11, wherein the protein having an activity of degrading a dsRNA is the protein defined by any one of claims 1 to 7.

19. The method according to claim 11, wherein the protein having an activity of degrading a dsRNA is a native Dicer or a functional equivalent thereof.

20. A method for synthesizing an RNA, the method comprising conducting an RNA synthesis reaction using a protein having an activity of synthesizing an RNA in the presence of a protein having an activity of binding to a nucleic acid.

21. The method according to claim 20, wherein a fusion protein of the protein having an activity of binding to a nucleic acid and the protein having an activity of synthesizing an RNA is used.

22. The method according to claim 20, wherein the protein having an activity of binding to a nucleic acid is a cold shock protein.

23. The method according to claim 22, wherein the cold shock protein is derived from a thermophilic bacterium or a thermostable bacterium.

24. The method according to claim 23, wherein the cold shock protein is cold shock protein B from *Thermotoga maritima.*

25. The method according to claim 20, wherein the protein having an activity of synthesizing an RNA is a DNA-dependent RNA polymerase.

26. A composition used for the method defined by claim 11, the composition containing a protein having an activity of binding to a nucleic acid and a protein having an activity of degrading a dsRNA.

27. A kit used for the method defined by claim 11, the kit containing a protein having an activity of binding to a nucleic acid and a protein having an activity of degrading a dsRNA.

28. A composition used for the method defined by claim 20, the composition containing a protein having an activity of binding to a nucleic acid and a protein having an activity of synthesizing an RNA.

29. A kit used for the method defined by claim 20, the kit containing a protein having an activity of binding to a nucleic acid and a protein having an activity of synthesizing an RNA.


SEQUENCE LISTING

<110>  TAKARA BIO INC.

<120>  Methods of degrading dsRNA and synthesizing RNA

<130>  38-90

<140>  EP 04 771 467.0
<141>  2004-08-10

<150>  JP 2003-293553
<151>  2003-08-14

<150>  JP 2003-342126
<151>  2003-09-30

<150>  JP 2003-409639
<151>  2003-12-08

<150>  JP 2004-086129
<151>  2004-03-24

<160>  27

<170>  PatentIn version 3.1

<210>  1
<211>  1924
<212>  PRT
<213>  Homo sapiens

<400>  1

<210>  2
<211>  5772
<212>  DNA
<213>  Homo sapiens

<400> 2
atgaaaagcc ctgctttgca acccctcagc atggcaggcc tgcagctcat gacccctgct 60
tcctcaccaa tgggtccttt ctttggactg ccatggcaac aagaagcaat tcatgataac 120
atttatacgc caagaaaata tcaggttgaa ctgcttgaag cagctctgga tcataatacc 180
atcgtctgtt taaacactgg ctcagggaag acatttattg ctagtactac tctactaaag 240
agctgtctct atctagatct aggggagact tcagctagaa atggaaaaag gacggtgttc 300
ttggtcaact ctgcaaacca ggttgctcaa caagtgtcag ctgtcagaac tcattcagat 360
ctcaaggttg gggaatactc aaacctagaa gtaaatgcat cttggacaaa agagagatgg 420
aaccaagagt ttactaagca ccaggttctc attatgactt gctatgtcgc cttgaatgtt 480
ttgaaaaatg gttacttatc actgtcagac attaaccttt tggtgtttga tgagtgtcat 540
cttgcaatcc tagaccaccc ctatcgagaa tttatgaagc tctgtgaaat ttgtccatca 600
tgtcctcgca ttttgggact aactgcttcc attttaaatg ggaaatggga tccagaggat 660
ttggaagaaa agtttcagaa actagagaaa attcttaaga gtaatgctga aactgcaact 720
gacctggtgg tcttagacag gtatacttct cagccatgtg agattgtggt ggattgtgga 780
ccatttactg acagaagtgg gctttatgaa agactgctga tggaattaga agaagcactt 840
aattttatca atgattgtaa tatatctgta cattcaaaag aaagagattc tactttaatt 900
tcgaaacaga tactatcaga ctgtcgtgcc gtattggtag ttctgggacc ctggtgtgca 960
gataaagtag ctggaatgat ggtaagagaa ctacagaaat acatcaaaca tgagcaagag 1020
gagctgcaca ggaaattttt attgtttaca gacactttcc taaggaaaat acatgcacta 1080
tgtgaagagc acttctcacc tgcctcactt gacctgaaat ttgtaactcc taaagtaatc 1140
aaactgctcg aaatcttacg caaatataaa ccatatgagc gacacagttt tgaaagcgtt 1200
gagtggtata ataatagaaa tcaggataat tatgtgtcat ggagtgattc tgaggatgat 1260
gatgaggatg aagaaattga agaaaaagag aagccagaga caaattttcc ttctcctttt 1320
accaacattt tgtgcggaat tatttttgtg gaaagaagat acacagcagt tgtcttaaac 1380
agattgataa aggaagctgg caaacaagat ccagagctgg cttatatcag tagcaatttc 1440
ataactggac atggcattgg gaagaatcag cctcgcaaca acacgatgga agcagaattc 1500
agaaaacagg aagaggtact taggaaattt cgagcacatg agaccaacct gcttattgca 1560
acaagtattg tagaagaggg tgttgatata ccaaaatgca acttggtggt tcgttttgat 1620
ttgcccacag aatatcgatc ctatgttcaa tctaaaggaa gagcaagggc acccatctct 1680
aattatataa tgttagcgga tacagacaaa ataaaaagtt ttgaagaaga ccttaaaacc 1740
tacaaagcta ttgaaaagat cttgagaaac aagtgttcca agtcggttga tactggtgag 1800
actgacattg atcctgtcat ggatgatgat cacgttttcc caccatatgt gttgaggcct 1860
gacgatggtg gtccacgagt cacaatcaac acggccattg gacacatcaa tagatactgt 1920
gctagattac caagtgatcc gtttactcat ctagctccta aatgcagaac ccgagagttg 1980
cctgatggta cattttattc aactctttat ctgccaatta actcacctct tcgagcctcc 2040

attgttggtc caccaatgag ctgtgtacga ttggctgaaa gagttgtcgc tctcatttgc 2100
tgtgagaaac tgcacaaaat tggcgaactg gatgaccatt tgatgccagt tgggaaagag 2160
actgttaaat atgaagagga gcttgatttg catgatgaag aagagaccag tgttccagga 2220
agaccaggtt ccacgaaacg aaggcagtgc tacccaaaag caattccaga gtgtttgagg 2280
gatagttatc ccagacctga tcagccctgt tacctgtatg tgataggaat ggttttaact 2340
acacctttac ctgatgaact caactttaga aggcggaagc tctatcctcc tgaagatacc 2400
acaagatgct ttggaatact gacggccaaa cccatacctc agattccaca ctttcctgtg 2460
tacacacgct ctggagaggt taccatatcc attgagttga agaagtctgg tttcatgttg 2520
tctctacaaa tgcttgagtt gattacaaga cttcaccagt atatattctc acatattctt 2580
cggcttgaaa aacctgcact agaatttaaa cctacagacg ctgattcagc atactgtgtt 2640
ctacctctta atgttgttaa tgactccagc actttggata ttgactttaa attcatggaa 2700
gatattgaga agtctgaagc tcgcataggc attcccagta caaagtatac aaaagaaaca 2760
ccctttgttt ttaaattaga agattaccaa gatgccgtta tcattccaag atatcgcaat 2820
tttgatcagc ctcatcgatt ttatgtagct gatgtgtaca ctgatcttac cccactcagt 2880
aaatttcctt cccctgagta tgaaactttt gcagaatatt ataaaacaaa gtacaacctt 2940
gacctaacca atctcaacca gccactgctg gatgtggacc acacatcttc aagacttaat 3000
cttttgacac ctcgacattt gaatcagaag gggaaagcgc ttcctttaag cagtgctgag 3060
aagaggaaag ccaaatggga aagtctgcag aataaacaga tactggttcc agaactctgt 3120
gctatacatc caattccagc atcactgtgg agaaaagctg tttgtctccc cagcatactt 3180
tatcgccttc actgcctttt gactgcagag gagctaagag cccagactgc cagcgatgct 3240
ggcgtgggag tcagatcact tcctgcggat tttagatacc ctaacttaga cttcgggtgg 3300
aaaaaatcta ttgacagcaa atctttcatc tcaatttcta actcctcttc agctgaaaat 3360
gataattact gtaagcacag cacaattgtc cctgaaaatg ctgcacatca aggtgctaat 3420
agaacctcct ctctagaaaa tcatgaccaa atgtctgtga actgcagaac gttgctcagc 3480
gagtcccctg gtaagctcca cgttgaagtt tcagcagatc ttacagcaat taatggtctt 3540
tcttacaatc aaaatctcgc caatggcagt tatgatttag ctaacagaga cttttgccaa 3600
ggaaatcagc taaattacta caagcaggaa atacccgtgc aaccaactac ctcatattcc 3660
attcagaatt tatacagtta cgagaaccag ccccagccca gcgatgaatg tactctcctg 3720
agtaataaat accttgatgg aaatgctaac aaatctacct cagatggaag tcctgtgatg 3780
gccgtaatgc ctggtacgac agacactatt caagtgctca agggcaggat ggattctgag 3840
cagagccctt ctattgggta ctcctcaagg actcttggcc ccaatcctgg acttattctt 3900
caggctttga ctctgtcaaa cgctagtgat ggatttaacc tggagcggct tgaaatgctt 3960
ggcgactcct ttttaaagca tgccatcacc acatatctat tttgcactta ccctgatgcg 4020
catgagggcc gcctttcata tatgagaagc aaaaaggtca gcaactgtaa tctgtatcgc 4080

cttggaaaaa agaagggact acccagccgc atggtggtgt caatatttga tccccctgtg 4140
aattggcttc ctcctggtta tgtagtaaat caagacaaaa gcaacacaga taaatgggaa 4200
aaagatgaaa tgacaaaaga ctgcatgctg gcgaatggca aactggatga ggattacgag 4260
gaggaggatg aggaggagga gagcctgatg tggagggctc cgaaggaaga ggctgactat 4320
gaagatgatt tcctggagta tgatcaggaa catatcagat ttatagataa tatgttaatg 4380
gggtcaggag cttttgtaaa gaaaatctct ctttctcctt tttcaaccac tgattctgca 4440
tatgaatgga aaatgcccaa aaaatcctcc ttaggtagta tgccattttc atcagatttt 4500
gaggattttg actacagctc ttgggatgca atgtgctatc tggatcctag caaagctgtt 4560
gaagaagatg actttgtggt ggggttctgg aatccatcag aagaaaactg tggtgttgac 4620
acgggaaagc agtccatttc ttacgacttg cacactgagc agtgtattgc tgacaaaagc 4680
atagcggact gtgtggaagc cctgctgggc tgctatttaa ccagctgtgg ggagagggct 4740
gctcagcttt tcctctgttc actggggctg aaggtgctcc cggtaattaa aaggactgat 4800
cgggaaaagg ccctgtgccc tactcgggag aatttcaaca gccaacaaaa gaacctttca 4860
gtgagctgtg ctgctgcttc tgtggccagt tcacgctctt ctgtattgaa agactcggaa 4920
tatggttgtt tgaagattcc accaagatgt atgtttgatc atccagatgc agataaaaca 4980
ctgaatcacc ttatatcggg gtttgaaaat tttgaaaaga aaatcaacta cagattcaag 5040
aataaggctt accttctcca ggcttttaca catgcctcct accactacaa tactatcact 5100
gattgttacc agcgcttaga attcctggga gatgcgattt tggactacct cataaccaag 5160
cacctttatg aagacccgcg gcagcactcc ccgggggtcc tgacagacct gcggtctgcc 5220
ctggtcaaca acaccatctt tgcatcgctg gctgtaaagt acgactacca caagtacttc 5280
aaagctgtct ctcctgagct cttccatgtc attgatgact ttgtgcagtt tcagcttgag 5340
aagaatgaaa tgcaaggaat ggattctgag cttaggagat ctgaggagga tgaagagaaa 5400
gaagaggata ttgaagttcc aaaggccatg ggggatattt ttgagtcgct tgctggtgcc 5460
atttacatgg atagtgggat gtcactggag acagtctggc aggtgtacta tcccatgatg 5520
cggccactaa tagaaaagtt ttctgcaaat gtaccccgtt cccctgtgcg agaattgctt 5580
gaaatggaac cagaaactgc caaatttagc ccggctgaga gaacttacga cgggaaggtc 5640
agagtcactg tggaagtagt aggaaagggg aaatttaaag gtgttggtcg aagttacagg 5700
attgccaaat ctgcagcagc aagaagagcc ctccgaagcc tcaaagctaa tcaacctcag 5760
gttcccaata gc 5772

<210> 3
<211> 1962
<212> DNA
<213> Artificial sequence

<220>
<223> A gene encoding human dicer mutant

<400> 3
caagtgctca agggcaggat ggattctgag cagagccctt ctattgggta ctcctcaagg 60
actcttggcc ccaatcctgg acttattctt caggctttga ctctgtcaaa cgctagtgat 120
ggatttaacc tggagcggct tgaaatgctt ggcgactcct ttttaaagca tgccatcacc 180
acatatctat tttgcactta ccctgatgcg catgagggcc gcctttcata tatgagaagc 240
aaaaaggtca gcaactgtaa tctgtatcgc cttggaaaaa agaagggact acccagccgc 300
atggtggtgt caatatttga tccccctgtg aattggcttc ctcctggtta tgtagtaaat 360
caagacaaaa gcaacacaga taaatgggaa aaagatgaaa tgacaaaaga ctgcatgctg 420
gcgaatggca aactggatga ggattacgag gaggaggatg aggaggagga gagcctgatg 480
tggagggctc cgaaggaaga ggctgactat gaagatgatt tcctggagta tgatcaggaa 540
catatcagat ttatagataa tatgttaatg gggtcaggag cttttgtaaa gaaaatctct 600
ctttctcctt tttcaaccac tgattctgca tatgaatgga aaatgcccaa aaaatcctcc 660
ttaggtagta tgccattttc atcagatttt gaggattttg actacagctc ttgggatgca 720
atgtgctatc tggatcctag caaagctgtt gaagaagatg actttgtggt ggggttctgg 780
aatccatcag aagaaaactg tggtgttgac acgggaaagc agtccatttc ttacgacttg 840
cacactgagc agtgtattgc tgacaaaagc atagcggact gtgtggaagc cctgctgggc 900
tgctatttaa ccagctgtgg ggagagggct gctcagcttt tcctctgttc actggggctg 960
aaggtgctcc cggtaattaa aaggactgat cgggaaaagg ccctgtgccc tactcgggag 1020
aatttcaaca gccaacaaaa gaacctttca gtgagctgtg ctgctgcttc tgtggccagt 1080
tcacgctctt ctgtattgaa agactcggaa tatggttgtt tgaagattcc accaagatgt 1140
atgtttgatc atccagatgc agataaaaca ctgaatcacc ttatatcggg gtttgaaaat 1200
tttgaaaaga aaatcaacta cagattcaag aataaggctt accttctcca ggcttttaca 1260
catgcctcct accactacaa tactatcact gattgttacc agcgcttaga attcctggga 1320
gatgcgattt tggactacct cataaccaag cacctttatg aagacccgcg gcagcactcc 1380
ccgggggtcc tgacagacct gcggtctgcc ctggtcaaca acaccatctt tgcatcgctg 1440
gctgtaaagt acgactacca caagtacttc aaagctgtct ctcctgagct cttccatgtc 1500
attgatgact ttgtgcagtt tcagcttgag aagaatgaaa tgcaaggaat ggattctgag 1560
cttaggagat ctgaggagga tgaagagaaa gaagaggata ttgaagttcc aaaggccatg 1620
ggggatattt ttgagtcgct tgctggtgcc atttacatgg atagtgggat gtcactggag 1680
acagtctggc aggtgtacta tcccatgatg cggccactaa tagaaaagtt ttctgcaaat 1740
gtaccccgtt cccctgtgcg agaattgctt gaaatggaac cagaaactgc caaatttagc 1800
ccggctgaga gaacttacga cgggaaggtc agagtcactg tggaagtagt aggaaagggg 1860
aaatttaaag gtgttggtcg aagttacagg attgccaaat ctgcagcagc aagaagagcc 1920
ctccgaagcc tcaaagctaa tcaacctcag gttcccaata gc 1962

<210>  4
<211>  654
<212>  PRT
<213>  Artificial sequence

<220>
<223>  An amino acid sequence of human dicer mutant.

<400>  4

Gln Val Leu Lys Gly Arg Met Asp Ser Glu Gln Ser Pro Ser Ile Gly
1               5                   10                  15

Tyr Ser Ser Arg Thr Leu Gly Pro Asn Pro Gly Leu Ile Leu Gln Ala
            20                  25                  30

Leu Thr Leu Ser Asn Ala Ser Asp Gly Phe Asn Leu Glu Arg Leu Glu
        35                  40                  45

Met Leu Gly Asp Ser Phe Leu Lys His Ala Ile Thr Thr Tyr Leu Phe
    50                  55                  60

Cys Thr Tyr Pro Asp Ala His Glu Gly Arg Leu Ser Tyr Met Arg Ser
65                  70                  75                  80

Lys Lys Val Ser Asn Cys Asn Leu Tyr Arg Leu Gly Lys Lys Lys Gly
                85                  90                  95

Leu Pro Ser Arg Met Val Val Ser Ile Phe Asp Pro Pro Val Asn Trp
            100                 105                 110

Leu Pro Pro Gly Tyr Val Val Asn Gln Asp Lys Ser Asn Thr Asp Lys
        115                 120                 125

Trp Glu Lys Asp Glu Met Thr Lys Asp Cys Met Leu Ala Asn Gly Lys
    130                 135                 140

Leu Asp Glu Asp Tyr Glu Glu Glu Asp Glu Glu Glu Glu Ser Leu Met
145                 150                 155                 160

Trp Arg Ala Pro Lys Glu Glu Ala Asp Tyr Glu Asp Asp Phe Leu Glu
                165                 170                 175

Tyr Asp Gln Glu His Ile Arg Phe Ile Asp Asn Met Leu Met Gly Ser
            180                 185                 190

Gly Ala Phe Val Lys Lys Ile Ser Leu Ser Pro Phe Ser Thr Thr Asp
        195                 200                 205

Ser Ala Tyr Glu Trp Lys Met Pro Lys Lys Ser Ser Leu Gly Ser Met
    210                 215                 220

Pro Phe Ser Ser Asp Phe Glu Asp Phe Asp Tyr Ser Ser Trp Asp Ala

225                 230                 235                 240

Met Cys Tyr Leu Asp Pro Ser Lys Ala Val Glu Glu Asp Asp Phe Val
                245                 250                 255

Val Gly Phe Trp Asn Pro Ser Glu Glu Asn Cys Gly Val Asp Thr Gly
            260                 265                 270

Lys Gln Ser Ile Ser Tyr Asp Leu His Thr Glu Gln Cys Ile Ala Asp
        275                 280                 285

Lys Ser Ile Ala Asp Cys Val Glu Ala Leu Leu Gly Cys Tyr Leu Thr
    290                 295                 300

Ser Cys Gly Glu Arg Ala Ala Gln Leu Phe Leu Cys Ser Leu Gly Leu
305                 310                 315                 320

Lys Val Leu Pro Val Ile Lys Arg Thr Asp Arg Glu Lys Ala Leu Cys
                325                 330                 335

Pro Thr Arg Glu Asn Phe Asn Ser Gln Gln Lys Asn Leu Ser Val Ser
            340                 345                 350

Cys Ala Ala Ala Ser Val Ala Ser Ser Arg Ser Ser Val Leu Lys Asp
        355                 360                 365

Ser Glu Tyr Gly Cys Leu Lys Ile Pro Pro Arg Cys Met Phe Asp His
    370                 375                 380

Pro Asp Ala Asp Lys Thr Leu Asn His Leu Ile Ser Gly Phe Glu Asn
385                 390                 395                 400

Phe Glu Lys Lys Ile Asn Tyr Arg Phe Lys Asn Lys Ala Tyr Leu Leu
                405                 410                 415

Gln Ala Phe Thr His Ala Ser Tyr His Tyr Asn Thr Ile Thr Asp Cys
            420                 425                 430

Tyr Gln Arg Leu Glu Phe Leu Gly Asp Ala Ile Leu Asp Tyr Leu Ile
        435                 440                 445

Thr Lys His Leu Tyr Glu Asp Pro Arg Gln His Ser Pro Gly Val Leu
    450                 455                 460

Thr Asp Leu Arg Ser Ala Leu Val Asn Asn Thr Ile Phe Ala Ser Leu
465                 470                 475                 480

Ala Val Lys Tyr Asp Tyr His Lys Tyr Phe Lys Ala Val Ser Pro Glu
                485                 490                 495

Leu Phe His Val Ile Asp Asp Phe Val Gln Phe Gln Leu Glu Lys Asn

500                 505                 510

Glu Met Gln Gly Met Asp Ser Glu Leu Arg Arg Ser Glu Glu Asp Glu
        515                 520                 525

Glu Lys Glu Glu Asp Ile Glu Val Pro Lys Ala Met Gly Asp Ile Phe
    530                 535                 540

Glu Ser Leu Ala Gly Ala Ile Tyr Met Asp Ser Gly Met Ser Leu Glu
545                 550                 555                 560

Thr Val Trp Gln Val Tyr Tyr Pro Met Met Arg Pro Leu Ile Glu Lys
                565                 570                 575

Phe Ser Ala Asn Val Pro Arg Ser Pro Val Arg Glu Leu Leu Glu Met
            580                 585                 590

Glu Pro Glu Thr Ala Lys Phe Ser Pro Ala Glu Arg Thr Tyr Asp Gly
        595                 600                 605

Lys Val Arg Val Thr Val Glu Val Val Gly Lys Gly Lys Phe Lys Gly
    610                 615                 620

Val Gly Arg Ser Tyr Arg Ile Ala Lys Ser Ala Ala Ala Arg Arg Ala
625                 630                 635                 640

Leu Arg Ser Leu Lys Ala Asn Gln Pro Gln Val Pro Asn Ser
                645                 650

<210>  5
<211>  36
<212>  DNA
<213>  Artificial sequence

<220>
<223>  Synthetic primer 1 to amplify a gene encoding human dicer

<400>  5
tcgagctcgg taccccaagt gctcaagggc aggatg                                36

<210>  6
<211>  36
<212>  DNA
<213>  Artificial sequence

<220>
<223>  Synthetic primer 2 to amplify a gene encoding human dicer

<400>  6
tatctagaaa gcttttagct attgggaacc tgaggt                                36

<210>  7
<211>  42
<212>  DNA
<213>  Artificial sequence

<220>
<223>  Synthetic primer 3 to amplify a gene encoding red-shifted green
fluorescence protein

<400>  7
gggtaatacg actcactata gggagaatgg ctagcaaagg ag                          42


<210>  8
<211>  42
<212>  DNA
<213>  Artificial sequence

<220>
<223>  Synthetic primer 4 to amplify a gene encoding red-shifted green
fluorescence protein

<400>  8
gggtaatacg actcactata gggagatcag ttgtacagtt ca                          42


<210>  9
<211>  66
<212>  PRT
<213>  Thermotoga maritima

<400>  9

Met Arg Gly Lys Val Lys Trp Phe Asp Ser Lys Lys Gly Tyr Gly Phe
1               5                   10                  15


Ile Thr Lys Asp Glu Gly Gly Asp Val Phe Val His Trp Ser Ala Ile
            20                  25                  30


Glu Met Glu Gly Phe Lys Thr Leu Lys Glu Gly Gln Val Val Glu Phe
        35                  40                  45


Glu Ile Gln Glu Gly Lys Lys Gly Pro Gln Ala Ala His Val Lys Val
    50                  55                  60


Val Glu
65


<210>  10
<211>  198
<212>  DNA
<213>  Thermotoga maritima

<400>  10
atgagaggaa aggttaagtg gttcgattcc aagaagggct acggattcat cacaaaggac      60

gaaggaggag acgtgttcgt acactggtca gccatcgaaa tggaaggttt caaaactctg     120

aaggaaggcc aggtcgtcga gttcgagatt caggaaggca agaaaggtcc acaggcagcg     180

cacgtgaaag tagttgag                                                   198


<210>  11
<211>  720
<212>  DNA
<213>  Artificial sequence

<220>
<223>  A gene encoding red-shifted green fluorescence protein.

<400>  11
atggctagca aaggagaaga actcttcact ggagttgtcc caattcttgt tgaattagat      60

ggtgatgtta acggccacaa gttctctgtc agtggagagg gtgaaggtga tgcaacatac     120

ggaaaactta ccctgaagtt catctgcact actggcaaac tgcctgttcc atggccaaca     180

ctagtcacta ctctgtgcta tggtgttcaa tgcttttcaa gatacccgga tcatatgaaa     240

cggcatgact ttttcaagag tgccatgccc gaaggttatg tacaggaaag gaccatcttc     300

ttcaaagatg acggcaacta caagacacgt gctgaagtca agtttgaagg tgataccctt     360

gttaatagaa tcgagttaaa aggtattgac ttcaaggaag atggaaacat tctgggacac     420

aaattggaat acaactataa ctcacacaat gtatacatca tggcagacaa acaaaagaat     480

ggaatcaaag tgaacttcaa gacccgccac aacattgaag atggaagcgt tcaactagca     540

gaccattatc aacaaaatac tccaattggc gatggccctg tccttttacc agacaaccat     600

tacctgtcca cacaatctgc cctttcgaaa gatcccaacg aaaagagaga ccacatggtc     660

cttcttgagt ttgtaacagc tgctgggatt acacatggca tggatgaact gtacaactga     720

<210>  12
<211>  675
<212>  PRT
<213>  Artificial sequence

<220>
<223>  An amino acid sequence of human dicer mutant

<400>  12

Met Asn His Lys Val His His His His His His Ile Glu Gly Arg Asn
1               5                   10                  15

Ser Ser Ser Val Pro Gln Val Leu Lys Gly Arg Met Asp Ser Glu Gln
            20                  25                  30

Ser Pro Ser Ile Gly Tyr Ser Ser Arg Thr Leu Gly Pro Asn Pro Gly
        35                  40                  45

Leu Ile Leu Gln Ala Leu Thr Leu Ser Asn Ala Ser Asp Gly Phe Asn
    50                  55                  60

Leu Glu Arg Leu Glu Met Leu Gly Asp Ser Phe Leu Lys His Ala Ile
65                  70                  75                  80

Thr Thr Tyr Leu Phe Cys Thr Tyr Pro Asp Ala His Glu Gly Arg Leu
                85                  90                  95

Ser Tyr Met Arg Ser Lys Lys Val Ser Asn Cys Asn Leu Tyr Arg Leu
            100                 105                 110

Gly Lys Lys Lys Gly Leu Pro Ser Arg Met Val Val Ser Ile Phe Asp
        115                 120                 125

Pro Pro Val Asn Trp Leu Pro Pro Gly Tyr Val Val Asn Gln Asp Lys
    130                 135                 140

Ser Asn Thr Asp Lys Trp Glu Lys Asp Glu Met Thr Lys Asp Cys Met
145                 150                 155                 160

Leu Ala Asn Gly Lys Leu Asp Glu Asp Tyr Glu Glu Glu Asp Glu Glu
                165                 170                 175

Glu Glu Ser Leu Met Trp Arg Ala Pro Lys Glu Glu Ala Asp Tyr Glu
            180                 185                 190

Asp Asp Phe Leu Glu Tyr Asp Gln Glu His Ile Arg Phe Ile Asp Asn
        195                 200                 205

Met Leu Met Gly Ser Gly Ala Phe Val Lys Lys Ile Ser Leu Ser Pro
    210                 215                 220

Phe Ser Thr Thr Asp Ser Ala Tyr Glu Trp Lys Met Pro Lys Lys Ser
225                 230                 235                 240

Ser Leu Gly Ser Met Pro Phe Ser Ser Asp Phe Glu Asp Phe Asp Tyr
                245                 250                 255

Ser Ser Trp Asp Ala Met Cys Tyr Leu Asp Pro Ser Lys Ala Val Glu
            260                 265                 270

Glu Asp Asp Phe Val Val Gly Phe Trp Asn Pro Ser Glu Glu Asn Cys
        275                 280                 285

Gly Val Asp Thr Gly Lys Gln Ser Ile Ser Tyr Asp Leu His Thr Glu
    290                 295                 300

Gln Cys Ile Ala Asp Lys Ser Ile Ala Asp Cys Val Glu Ala Leu Leu
305                 310                 315                 320

Gly Cys Tyr Leu Thr Ser Cys Gly Glu Arg Ala Ala Gln Leu Phe Leu
                325                 330                 335

Cys Ser Leu Gly Leu Lys Val Leu Pro Val Ile Lys Arg Thr Asp Arg
            340                 345                 350

Glu Lys Ala Leu Cys Pro Thr Arg Glu Asn Phe Asn Ser Gln Gln Lys
        355                 360                 365

Asn Leu Ser Val Ser Cys Ala Ala Ala Ser Val Ala Ser Ser Arg Ser
    370                 375                 380

Ser Val Leu Lys Asp Ser Glu Tyr Gly Cys Leu Lys Ile Pro Pro Arg
385                 390                 395                 400

Cys Met Phe Asp His Pro Asp Ala Asp Lys Thr Leu Asn His Leu Ile
                405                 410                 415

Ser Gly Phe Glu Asn Phe Glu Lys Lys Ile Asn Tyr Arg Phe Lys Asn
            420                 425                 430

Lys Ala Tyr Leu Leu Gln Ala Phe Thr His Ala Ser Tyr His Tyr Asn
        435                 440                 445

Thr Ile Thr Asp Cys Tyr Gln Arg Leu Glu Phe Leu Gly Asp Ala Ile
    450                 455                 460

Leu Asp Tyr Leu Ile Thr Lys His Leu Tyr Glu Asp Pro Arg Gln His
465                 470                 475                 480

Ser Pro Gly Val Leu Thr Asp Leu Arg Ser Ala Leu Val Asn Asn Thr
                485                 490                 495

Ile Phe Ala Ser Leu Ala Val Lys Tyr Asp Tyr His Lys Tyr Phe Lys
            500                 505                 510

Ala Val Ser Pro Glu Leu Phe His Val Ile Asp Asp Phe Val Gln Phe
        515                 520                 525

Gln Leu Glu Lys Asn Glu Met Gln Gly Met Asp Ser Glu Leu Arg Arg
    530                 535                 540

Ser Glu Glu Asp Glu Glu Lys Glu Glu Asp Ile Glu Val Pro Lys Ala
545                 550                 555                 560

Met Gly Asp Ile Phe Glu Ser Leu Ala Gly Ala Ile Tyr Met Asp Ser
                565                 570                 575

Gly Met Ser Leu Glu Thr Val Trp Gln Val Tyr Tyr Pro Met Met Arg
            580                 585                 590

Pro Leu Ile Glu Lys Phe Ser Ala Asn Val Pro Arg Ser Pro Val Arg
        595                 600                 605

Glu Leu Leu Glu Met Glu Pro Glu Thr Ala Lys Phe Ser Pro Ala Glu
    610                 615                 620

Arg Thr Tyr Asp Gly Lys Val Arg Val Thr Val Glu Val Val Gly Lys
625                 630                 635                 640

Gly Lys Phe Lys Gly Val Gly Arg Ser Tyr Arg Ile Ala Lys Ser Ala
                645                 650                 655

Ala Ala Arg Arg Ala Leu Arg Ser Leu Lys Ala Asn Gln Pro Gln Val
        660                 665                 670

Pro Asn Ser
    675

<210> 13
<211> 2025
<212> DNA
<213> Artificial sequence

<220>
<223> A gene encoding human dicer mutant

<400> 13
   atgaatcaca aagtgcatca tcatcatcat catatcgaag gtaggaattc gagctcggta      60
   ccccaagtgc tcaagggcag gatggattct gagcagagcc cttctattgg gtactcctca     120
   aggactcttg gccccaatcc tggacttatt cttcaggctt tgactctgtc aaacgctagt     180
   gatggattta acctggagcg gcttgaaatg cttggcgact cctttttaaa gcatgccatc     240
   accacatatc tattttgcac ttaccctgat gcgcatgagg gccgcctttc atatatgaga     300
   agcaaaaagg tcagcaactg taatctgtat cgccttggaa aaaagaaggg actacccagc     360
   cgcatggtgg tgtcaatatt tgatccccct gtgaattggc ttcctcctgg ttatgtagta     420
   aatcaagaca aaagcaacac agataaatgg gaaaaagatg aaatgacaaa agactgcatg     480
   ctggcgaatg gcaaactgga tgaggattac gaggaggagg atgaggagga ggagagcctg     540
   atgtggaggg ctccgaagga agaggctgac tatgaagatg atttcctgga gtatgatcag     600
   gaacatatca gatttataga taatatgtta atggggtcag gagcttttgt aaagaaaatc     660
   tctctttctc ctttttcaac cactgattct gcatatgaat ggaaaatgcc caaaaaatcc     720
   tccttaggta gtatgccatt ttcatcagat tttgaggatt ttgactacag ctcttgggat     780
   gcaatgtgct atctggatcc tagcaaagct gttgaagaag atgactttgt ggtggggttc     840
   tggaatccat cagaagaaaa ctgtggtgtt gacacgggaa agcagtccat ttcttacgac     900
   ttgcacactg agcagtgtat tgctgacaaa agcatagcgg actgtgtgga agccctgctg     960
   ggctgctatt taaccagctg tggggagagg gctgctcagc ttttcctctg ttcactgggg    1020
   ctgaaggtgc tcccggtaat taaaaggact gatcgggaaa aggccctgtg ccctactcgg    1080
   gagaatttca acagccaaca aaagaacctt tcagtgagct gtgctgctgc ttctgtggcc    1140
   agttcacgct cttctgtatt gaaagactcg gaatatggtt gtttgaagat tccaccaaga    1200
   tgtatgtttg atcatccaga tgcagataaa acactgaatc accttatatc ggggtttgaa    1260
   aattttgaaa agaaaatcaa ctacagattc aagaataagg cttaccttct ccaggctttt    1320
   acacatgcct cctaccacta caatactatc actgattgtt accagcgctt agaattcctg    1380
   ggagatgcga ttttggacta cctcataacc aagcaccttt atgaagaccc gcggcagcac    1440
   tccccggggg tcctgacaga cctgcggtct gccctggtca acaacaccat ctttgcatcg    1500
   ctggctgtaa agtacgacta ccacaagtac ttcaaagctg tctctcctga gctcttccat    1560
   gtcattgatg actttgtgca gtttcagctt gagaagaatg aaatgcaagg aatggattct 1620
   gagcttagga gatctgagga ggatgaagag aaagaagagg atattgaagt tccaaaggcc 1680
   atgggggata tttttgagtc gcttgctggt gccatttaca tggatagtgg gatgtcactg 1740
   gagacagtct ggcaggtgta ctatcccatg atgcggccac taatagaaaa gttttctgca 1800
   aatgtacccc gttcccctgt gcgagaattg cttgaaatgg aaccagaaac tgccaaattt 1860
   agcccggctg agagaactta cgacgggaag gtcagagtca ctgtggaagt agtaggaaag 1920
   gggaaattta aaggtgttgg tcgaagttac aggattgcca aatctgcagc agcaagaaga 1980
   gccctccgaa gcctcaaagc taatcaacct caggttccca atagc 2025

<210> 14
<211> 36
<212> DNA
<213> Artificial

<220>
<223> Synthetic primer 5 to amplify a gene encoding human dicer mutant

<400> 14
tcgagctcgg tacccgcctc cattgttggt ccacca 36

<210> 15
<211> 36
<212> DNA
<213> Artificial

<220>
<223> Synthetic primer 6 to amplify a gene encoding human dicer mutant

<400> 15
tatctagaaa gcttttagct attgggaacc tgaggt 36

<210> 16
<211> 3741
<212> DNA
<213> Artificial

<220>
<223> A gene encoding human dicer mutant

<400> 16
gcctccattg ttggtccacc aatgagctgt gtacgattgg ctgaaagagt tgtcgctctc 60

atttgctgtg agaaactgca caaaattggc gaactggatg accatttgat gccagttggg 120

aaagagactg ttaaatatga agaggagctt gatttgcatg atgaagaaga gaccagtgtt 180

ccaggaagac caggttccac gaaacgaagg cagtgctacc caaaagcaat tccagagtgt 240

ttgagggata gttatcccag acctgatcag ccctgttacc tgtatgtgat aggaatggtt 300

ttaactacac ctttacctga tgaactcaac tttagaaggc ggaagctcta tcctcctgaa 360

gataccacaa gatgctttgg aatactgacg gccaaaccca tacctcagat tccacacttt 420

cctgtgtaca cacgctctgg agaggttacc atatccattg agttgaagaa gtctggtttc 480

atgttgtctc tacaaatgct tgagttgatt acaagacttc accagtatat attctcacat 540

attcttcggc ttgaaaaacc tgcactagaa tttaaaccta cagacgctga ttcagcatac 600
tgtgttctac ctcttaatgt tgttaatgac tccagcactt tggatattga ctttaaattc 660
atggaagata ttgagaagtc tgaagctcgc ataggcattc ccagtacaaa gtatacaaaa 720
gaaacaccct ttgtttttaa attagaagat taccaagatg ccgttatcat tccaagatat 780
cgcaattttg atcagcctca tcgattttat gtagctgatg tgtacactga tcttacccca 840
ctcagtaaat ttccttcccc tgagtatgaa acttttgcag aatattataa aacaaagtac 900
aaccttgacc taaccaatct caaccagcca ctgctggatg tggaccacac atcttcaaga 960
cttaatcttt tgacacctcg acatttgaat cagaagggga aagcgcttcc tttaagcagt 1020
gctgagaaga ggaaagccaa atgggaaagt ctgcagaata aacagatact ggttccagaa 1080
ctctgtgcta tacatccaat tccagcatca ctgtggagaa aagctgtttg tctccccagc 1140
atactttatc gccttcactg ccttttgact gcagaggagc taagagccca gactgccagc 1200
gatgctggcg tgggagtcag atcacttcct gcggatttta gataccctaa cttagacttc 1260
gggtggaaaa aatctattga cagcaaatct ttcatctcaa tttctaactc ctcttcagct 1320
gaaaatgata attactgtaa gcacagcaca attgtccctg aaaatgctgc acatcaaggt 1380
gctaatagaa cctcctctct agaaaatcat gaccaaatgt ctgtgaactg cagaacgttg 1440
ctcagcgagt cccctggtaa gctccacgtt gaagtttcag cagatcttac agcaattaat 1500
ggtctttctt acaatcaaaa tctcgccaat ggcagttatg atttagctaa cagagacttt 1560
tgccaaggaa atcagctaaa ttactacaag caggaaatac ccgtgcaacc aactacctca 1620
tattccattc agaatttata cagttacgag aaccagcccc agcccagcga tgaatgtact 1680
ctcctgagta ataaatacct tgatggaaat gctaacaaat ctacctcaga tggaagtcct 1740
gtgatggccg taatgcctgg tacgacagac actattcaag tgctcaaggg caggatggat 1800
tctgagcaga gcccttctat tgggtactcc tcaaggactc ttggccccaa tcctggactt 1860
attcttcagg ctttgactct gtcaaacgct agtgatggat ttaacctgga gcggcttgaa 1920
atgcttggcg actccttttt aaagcatgcc atcaccacat atctattttg cacttaccct 1980
gatgcgcatg agggccgcct ttcatatatg agaagcaaaa aggtcagcaa ctgtaatctg 2040
tatcgccttg gaaaaaagaa gggactaccc agccgcatgg tggtgtcaat atttgatccc 2100
cctgtgaatt ggcttcctcc tggttatgta gtaaatcaag acaaaagcaa cacagataaa 2160
tgggaaaaag atgaaatgac aaaagactgc atgctggcga atggcaaact ggatgaggat 2220
tacgaggagg aggatgagga ggaggagagc ctgatgtgga gggctccgaa ggaagaggct 2280
gactatgaag atgatttcct ggagtatgat caggaacata tcagatttat agataatatg 2340
ttaatggggt caggagcttt tgtaaagaaa atctctcttt ctcctttttc aaccactgat 2400
tctgcatatg aatggaaaat gcccaaaaaa tcctccttag gtagtatgcc attttcatca 2460
gattttgagg attttgacta cagctcttgg gatgcaatgt gctatctgga tcctagcaaa 2520
gctgttgaag aagatgactt tgtggtgggg ttctggaatc catcagaaga aaactgtggt 2580

gttgacacgg gaaagcagtc catttcttac gacttgcaca ctgagcagtg tattgctgac 2640

aaaagcatag cggactgtgt ggaagccctg ctgggctgct atttaaccag ctgtggggag 2700

agggctgctc agcttttcct ctgttcactg gggctgaagg tgctcccggt aattaaaagg 2760

actgatcggg aaaaggccct gtgccctact cgggagaatt tcaacagcca acaaaagaac 2820

ctttcagtga gctgtgctgc tgcttctgtg gccagttcac gctcttctgt attgaaagac 2880

tcggaatatg gttgtttgaa gattccacca agatgtatgt ttgatcatcc agatgcagat 2940

aaaacactga atcaccttat atcggggttt gaaaattttg aaaagaaaat caactacaga 3000

ttcaagaata aggcttacct tctccaggct tttacacatg cctcctacca ctacaatact 3060

atcactgatt gttaccagcg cttagaattc ctgggagatg cgattttgga ctacctcata 3120

accaagcacc tttatgaaga cccgcggcag cactccccgg gggtcctgac agacctgcgg 3180

tctgccctgg tcaacaacac catctttgca tcgctggctg taaagtacga ctaccacaag 3240

tacttcaaag ctgtctctcc tgagctcttc catgtcattg atgactttgt gcagtttcag 3300

cttgagaaga atgaaatgca aggaatggat tctgagctta ggagatctga ggaggatgaa 3360

gagaaagaag aggatattga agttccaaag gccatggggg atatttttga gtcgcttgct 3420

ggtgccattt acatggatag tgggatgtca ctggagacag tctggcaggt gtactatccc 3480

atgatgcggc cactaataga aaagttttct gcaaatgtac cccgttcccc tgtgcgagaa 3540

ttgcttgaaa tggaaccaga aactgccaaa tttagcccgg ctgagagaac ttacgacggg 3600

aaggtcagag tcactgtgga agtagtagga aaggggaaat ttaaaggtgt tggtcgaagt 3660

tacaggattg ccaaatctgc agcagcaaga agagccctcc gaagcctcaa agctaatcaa 3720

cctcaggttc ccaatagcta a 3741

<210> 17
<211> 1246
<212> PRT
<213> Artificial

<220>
<223> An amino acid sequence of human dicer mutant

<400> 17

Ala Ser Ile Val Gly Pro Pro Met Ser Cys Val Arg Leu Ala Glu Arg
1               5                   10                  15

Val Val Ala Leu Ile Cys Cys Glu Lys Leu His Lys Ile Gly Glu Leu
            20                  25                  30

Asp Asp His Leu Met Pro Val Gly Lys Glu Thr Val Lys Tyr Glu Glu
        35                  40                  45

Glu Leu Asp Leu His Asp Glu Glu Glu Thr Ser Val Pro Gly Arg Pro
    50                  55                  60

Gly Ser Thr Lys Arg Arg Gln Cys Tyr Pro Lys Ala Ile Pro Glu Cys

65                  70                  75                  80

Leu Arg Asp Ser Tyr Pro Arg Pro Asp Gln Pro Cys Tyr Leu Tyr Val
                85                  90                  95

Ile Gly Met Val Leu Thr Thr Pro Leu Pro Asp Glu Leu Asn Phe Arg
            100                 105                 110

Arg Arg Lys Leu Tyr Pro Pro Glu Asp Thr Thr Arg Cys Phe Gly Ile
        115                 120                 125

Leu Thr Ala Lys Pro Ile Pro Gln Ile Pro His Phe Pro Val Tyr Thr
    130                 135                 140

Arg Ser Gly Glu Val Thr Ile Ser Ile Glu Leu Lys Lys Ser Gly Phe
145                 150                 155                 160

Met Leu Ser Leu Gln Met Leu Glu Leu Ile Thr Arg Leu His Gln Tyr
                165                 170                 175

Ile Phe Ser His Ile Leu Arg Leu Glu Lys Pro Ala Leu Glu Phe Lys
            180                 185                 190

Pro Thr Asp Ala Asp Ser Ala Tyr Cys Val Leu Pro Leu Asn Val Val
        195                 200                 205

Asn Asp Ser Ser Thr Leu Asp Ile Asp Phe Lys Phe Met Glu Asp Ile
    210                 215                 220

Glu Lys Ser Glu Ala Arg Ile Gly Ile Pro Ser Thr Lys Tyr Thr Lys
225                 230                 235                 240

Glu Thr Pro Phe Val Phe Lys Leu Glu Asp Tyr Gln Asp Ala Val Ile
                245                 250                 255

Ile Pro Arg Tyr Arg Asn Phe Asp Gln Pro His Arg Phe Tyr Val Ala
            260                 265                 270

Asp Val Tyr Thr Asp Leu Thr Pro Leu Ser Lys Phe Pro Ser Pro Glu
        275                 280                 285

Tyr Glu Thr Phe Ala Glu Tyr Tyr Lys Thr Lys Tyr Asn Leu Asp Leu
    290                 295                 300

Thr Asn Leu Asn Gln Pro Leu Leu Asp Val Asp His Thr Ser Ser Arg
305                 310                 315                 320

Leu Asn Leu Leu Thr Pro Arg His Leu Asn Gln Lys Gly Lys Ala Leu
                325                 330                 335

Pro Leu Ser Ser Ala Glu Lys Arg Lys Ala Lys Trp Glu Ser Leu Gln

            340                 345                 350

Asn Lys Gln Ile Leu Val Pro Glu Leu Cys Ala Ile His Pro Ile Pro
        355                 360                 365

Ala Ser Leu Trp Arg Lys Ala Val Cys Leu Pro Ser Ile Leu Tyr Arg
    370                 375                 380

Leu His Cys Leu Leu Thr Ala Glu Glu Leu Arg Ala Gln Thr Ala Ser
385                 390                 395                 400

Asp Ala Gly Val Gly Val Arg Ser Leu Pro Ala Asp Phe Arg Tyr Pro
                405                 410                 415

Asn Leu Asp Phe Gly Trp Lys Lys Ser Ile Asp Ser Lys Ser Phe Ile
            420                 425                 430

Ser Ile Ser Asn Ser Ser Ser Ala Glu Asn Asp Asn Tyr Cys Lys His
        435                 440                 445

Ser Thr Ile Val Pro Glu Asn Ala Ala His Gln Gly Ala Asn Arg Thr
    450                 455                 460

Ser Ser Leu Glu Asn His Asp Gln Met Ser Val Asn Cys Arg Thr Leu
465                 470                 475                 480

Leu Ser Glu Ser Pro Gly Lys Leu His Val Glu Val Ser Ala Asp Leu
                485                 490                 495

Thr Ala Ile Asn Gly Leu Ser Tyr Asn Gln Asn Leu Ala Asn Gly Ser
            500                 505                 510

Tyr Asp Leu Ala Asn Arg Asp Phe Cys Gln Gly Asn Gln Leu Asn Tyr
        515                 520                 525

Tyr Lys Gln Glu Ile Pro Val Gln Pro Thr Thr Ser Tyr Ser Ile Gln
    530                 535                 540

Asn Leu Tyr Ser Tyr Glu Asn Gln Pro Gln Pro Ser Asp Glu Cys Thr
545                 550                 555                 560

Leu Leu Ser Asn Lys Tyr Leu Asp Gly Asn Ala Asn Lys Ser Thr Ser
                565                 570                 575

Asp Gly Ser Pro Val Met Ala Val Met Pro Gly Thr Thr Asp Thr Ile
            580                 585                 590

Gln Val Leu Lys Gly Arg Met Asp Ser Glu Gln Ser Pro Ser Ile Gly
        595                 600                 605

Tyr Ser Ser Arg Thr Leu Gly Pro Asn Pro Gly Leu Ile Leu Gln Ala

610                 615                 620

Leu Thr Leu Ser Asn Ala Ser Asp Gly Phe Asn Leu Glu Arg Leu Glu
625                 630                 635                 640

Met Leu Gly Asp Ser Phe Leu Lys His Ala Ile Thr Thr Tyr Leu Phe
                645                 650                 655

Cys Thr Tyr Pro Asp Ala His Glu Gly Arg Leu Ser Tyr Met Arg Ser
            660                 665                 670

Lys Lys Val Ser Asn Cys Asn Leu Tyr Arg Leu Gly Lys Lys Lys Gly
        675                 680                 685

Leu Pro Ser Arg Met Val Val Ser Ile Phe Asp Pro Pro Val Asn Trp
    690                 695                 700

Leu Pro Pro Gly Tyr Val Val Asn Gln Asp Lys Ser Asn Thr Asp Lys
705                 710                 715                 720

Trp Glu Lys Asp Glu Met Thr Lys Asp Cys Met Leu Ala Asn Gly Lys
                725                 730                 735

Leu Asp Glu Asp Tyr Glu Glu Glu Asp Glu Glu Glu Glu Ser Leu Met
            740                 745                 750

Trp Arg Ala Pro Lys Glu Glu Ala Asp Tyr Glu Asp Asp Phe Leu Glu
        755                 760                 765

Tyr Asp Gln Glu His Ile Arg Phe Ile Asp Asn Met Leu Met Gly Ser
    770                 775                 780

Gly Ala Phe Val Lys Lys Ile Ser Leu Ser Pro Phe Ser Thr Thr Asp
785                 790                 795                 800

Ser Ala Tyr Glu Trp Lys Met Pro Lys Lys Ser Ser Leu Gly Ser Met
                805                 810                 815

Pro Phe Ser Ser Asp Phe Glu Asp Phe Asp Tyr Ser Ser Trp Asp Ala
            820                 825                 830

Met Cys Tyr Leu Asp Pro Ser Lys Ala Val Glu Glu Asp Asp Phe Val
        835                 840                 845

Val Gly Phe Trp Asn Pro Ser Glu Glu Asn Cys Gly Val Asp Thr Gly
    850                 855                 860

Lys Gln Ser Ile Ser Tyr Asp Leu His Thr Glu Gln Cys Ile Ala Asp
865                 870                 875                 880

Lys Ser Ile Ala Asp Cys Val Glu Ala Leu Leu Gly Cys Tyr Leu Thr

                885                 890                 895

Ser Cys Gly Glu Arg Ala Ala Gln Leu Phe Leu Cys Ser Leu Gly Leu
            900                 905                 910

Lys Val Leu Pro Val Ile Lys Arg Thr Asp Arg Glu Lys Ala Leu Cys
        915                 920                 925

Pro Thr Arg Glu Asn Phe Asn Ser Gln Gln Lys Asn Leu Ser Val Ser
    930                 935                 940

Cys Ala Ala Ala Ser Val Ala Ser Ser Arg Ser Ser Val Leu Lys Asp
945                 950                 955                 960

Ser Glu Tyr Gly Cys Leu Lys Ile Pro Pro Arg Cys Met Phe Asp His
                965                 970                 975

Pro Asp Ala Asp Lys Thr Leu Asn His Leu Ile Ser Gly Phe Glu Asn
            980                 985                 990

Phe Glu Lys Lys Ile Asn Tyr Arg Phe Lys Asn Lys Ala Tyr Leu Leu
        995                 1000                1005

Gln Ala Phe Thr His Ala Ser Tyr His Tyr Asn Thr Ile Thr Asp
    1010                1015                1020

Cys Tyr Gln Arg Leu Glu Phe Leu Gly Asp Ala Ile Leu Asp Tyr
    1025                1030                1035

Leu Ile Thr Lys His Leu Tyr Glu Asp Pro Arg Gln His Ser Pro
    1040                1045                1050

Gly Val Leu Thr Asp Leu Arg Ser Ala Leu Val Asn Asn Thr Ile
    1055                1060                1065

Phe Ala Ser Leu Ala Val Lys Tyr Asp Tyr His Lys Tyr Phe Lys
    1070                1075                1080

Ala Val Ser Pro Glu Leu Phe His Val Ile Asp Asp Phe Val Gln
    1085                1090                1095

Phe Gln Leu Glu Lys Asn Glu Met Gln Gly Met Asp Ser Glu Leu
    1100                1105                1110

Arg Arg Ser Glu Glu Asp Glu Glu Lys Glu Glu Asp Ile Glu Val
    1115                1120                1125

Pro Lys Ala Met Gly Asp Ile Phe Glu Ser Leu Ala Gly Ala Ile
    1130                1135                1140

Tyr Met Asp Ser Gly Met Ser Leu Glu Thr Val Trp Gln Val Tyr

1145                1150                1155

Tyr Pro Met Met Arg Pro Leu Ile Glu Lys Phe Ser Ala Asn Val
    1160                1165                1170

Pro Arg Ser Pro Val Arg Glu Leu Leu Glu Met Glu Pro Glu Thr
    1175                1180                1185

Ala Lys Phe Ser Pro Ala Glu Arg Thr Tyr Asp Gly Lys Val Arg
    1190                1195                1200

Val Thr Val Glu Val Val Gly Lys Gly Lys Phe Lys Gly Val Gly
    1205                1210                1215

Arg Ser Tyr Arg Ile Ala Lys Ser Ala Ala Ala Arg Arg Ala Leu
    1220                1225                1230

Arg Ser Leu Lys Ala Asn Gln Pro Gln Val Pro Asn Ser
    1235                1240                1245

<210>  18
<211>  1267
<212>  PRT
<213>  Artificial

<220>
<223>  An amino acid sequence of human dicer mutant

<400>  18

Met Asn His Lys Val His His His His His His Ile Glu Gly Arg Asn
1               5                   10                  15

Ser Ser Ser Val Pro Ala Ser Ile Val Gly Pro Pro Met Ser Cys Val
            20                  25                  30

Arg Leu Ala Glu Arg Val Val Ala Leu Ile Cys Cys Glu Lys Leu His
        35                  40                  45

Lys Ile Gly Glu Leu Asp Asp His Leu Met Pro Val Gly Lys Glu Thr
    50                  55                  60

Val Lys Tyr Glu Glu Glu Leu Asp Leu His Asp Glu Glu Glu Thr Ser
65                  70                  75                  80

Val Pro Gly Arg Pro Gly Ser Thr Lys Arg Arg Gln Cys Tyr Pro Lys
                85                  90                  95

Ala Ile Pro Glu Cys Leu Arg Asp Ser Tyr Pro Arg Pro Asp Gln Pro
            100                 105                 110

Cys Tyr Leu Tyr Val Ile Gly Met Val Leu Thr Thr Pro Leu Pro Asp
        115                 120                 125

Glu Leu Asn Phe Arg Arg Arg Lys Leu Tyr Pro Pro Glu Asp Thr Thr
    130                 135                 140

Arg Cys Phe Gly Ile Leu Thr Ala Lys Pro Ile Pro Gln Ile Pro His
145                 150                 155                 160

Phe Pro Val Tyr Thr Arg Ser Gly Glu Val Thr Ile Ser Ile Glu Leu
                165                 170                 175

Lys Lys Ser Gly Phe Met Leu Ser Leu Gln Met Leu Glu Leu Ile Thr
            180                 185                 190

Arg Leu His Gln Tyr Ile Phe Ser His Ile Leu Arg Leu Glu Lys Pro
        195                 200                 205

Ala Leu Glu Phe Lys Pro Thr Asp Ala Asp Ser Ala Tyr Cys Val Leu
    210                 215                 220

Pro Leu Asn Val Val Asn Asp Ser Ser Thr Leu Asp Ile Asp Phe Lys
225                 230                 235                 240

Phe Met Glu Asp Ile Glu Lys Ser Glu Ala Arg Ile Gly Ile Pro Ser
                245                 250                 255

Thr Lys Tyr Thr Lys Glu Thr Pro Phe Val Phe Lys Leu Glu Asp Tyr
            260                 265                 270

Gln Asp Ala Val Ile Ile Pro Arg Tyr Arg Asn Phe Asp Gln Pro His
        275                 280                 285

Arg Phe Tyr Val Ala Asp Val Tyr Thr Asp Leu Thr Pro Leu Ser Lys
    290                 295                 300

Phe Pro Ser Pro Glu Tyr Glu Thr Phe Ala Glu Tyr Tyr Lys Thr Lys
305                 310                 315                 320

Tyr Asn Leu Asp Leu Thr Asn Leu Asn Gln Pro Leu Leu Asp Val Asp
                325                 330                 335

His Thr Ser Ser Arg Leu Asn Leu Leu Thr Pro Arg His Leu Asn Gln
            340                 345                 350

Lys Gly Lys Ala Leu Pro Leu Ser Ser Ala Glu Lys Arg Lys Ala Lys
        355                 360                 365

Trp Glu Ser Leu Gln Asn Lys Gln Ile Leu Val Pro Glu Leu Cys Ala
    370                 375                 380

Ile His Pro Ile Pro Ala Ser Leu Trp Arg Lys Ala Val Cys Leu Pro
385                 390                 395                 400

Ser Ile Leu Tyr Arg Leu His Cys Leu Leu Thr Ala Glu Glu Leu Arg
                405                 410                 415

Ala Gln Thr Ala Ser Asp Ala Gly Val Gly Val Arg Ser Leu Pro Ala
            420                 425                 430

Asp Phe Arg Tyr Pro Asn Leu Asp Phe Gly Trp Lys Lys Ser Ile Asp
        435                 440                 445

Ser Lys Ser Phe Ile Ser Ile Ser Asn Ser Ser Ser Ala Glu Asn Asp
    450                 455                 460

Asn Tyr Cys Lys His Ser Thr Ile Val Pro Glu Asn Ala Ala His Gln
465                 470                 475                 480

Gly Ala Asn Arg Thr Ser Ser Leu Glu Asn His Asp Gln Met Ser Val
                485                 490                 495

Asn Cys Arg Thr Leu Leu Ser Glu Ser Pro Gly Lys Leu His Val Glu
            500                 505                 510

Val Ser Ala Asp Leu Thr Ala Ile Asn Gly Leu Ser Tyr Asn Gln Asn
        515                 520                 525

Leu Ala Asn Gly Ser Tyr Asp Leu Ala Asn Arg Asp Phe Cys Gln Gly
    530                 535                 540

Asn Gln Leu Asn Tyr Tyr Lys Gln Glu Ile Pro Val Gln Pro Thr Thr
545                 550                 555                 560

Ser Tyr Ser Ile Gln Asn Leu Tyr Ser Tyr Glu Asn Gln Pro Gln Pro
                565                 570                 575

Ser Asp Glu Cys Thr Leu Leu Ser Asn Lys Tyr Leu Asp Gly Asn Ala
            580                 585                 590

Asn Lys Ser Thr Ser Asp Gly Ser Pro Val Met Ala Val Met Pro Gly
        595                 600                 605

Thr Thr Asp Thr Ile Gln Val Leu Lys Gly Arg Met Asp Ser Glu Gln
    610                 615                 620

Ser Pro Ser Ile Gly Tyr Ser Ser Arg Thr Leu Gly Pro Asn Pro Gly
625                 630                 635                 640

Leu Ile Leu Gln Ala Leu Thr Leu Ser Asn Ala Ser Asp Gly Phe Asn
                645                 650                 655

Leu Glu Arg Leu Glu Met Leu Gly Asp Ser Phe Leu Lys His Ala Ile
            660                 665                 670

Thr Thr Tyr Leu Phe Cys Thr Tyr Pro Asp Ala His Glu Gly Arg Leu
        675                 680                 685

Ser Tyr Met Arg Ser Lys Lys Val Ser Asn Cys Asn Leu Tyr Arg Leu
    690                 695                 700

Gly Lys Lys Lys Gly Leu Pro Ser Arg Met Val Val Ser Ile Phe Asp
705                 710                 715                 720

Pro Pro Val Asn Trp Leu Pro Pro Gly Tyr Val Val Asn Gln Asp Lys
                725                 730                 735

Ser Asn Thr Asp Lys Trp Glu Lys Asp Glu Met Thr Lys Asp Cys Met
            740                 745                 750

Leu Ala Asn Gly Lys Leu Asp Glu Asp Tyr Glu Glu Glu Asp Glu Glu
        755                 760                 765

Glu Glu Ser Leu Met Trp Arg Ala Pro Lys Glu Glu Ala Asp Tyr Glu
    770                 775                 780

Asp Asp Phe Leu Glu Tyr Asp Gln Glu His Ile Arg Phe Ile Asp Asn
785                 790                 795                 800

Met Leu Met Gly Ser Gly Ala Phe Val Lys Lys Ile Ser Leu Ser Pro
                805                 810                 815

Phe Ser Thr Thr Asp Ser Ala Tyr Glu Trp Lys Met Pro Lys Lys Ser
            820                 825                 830

Ser Leu Gly Ser Met Pro Phe Ser Ser Asp Phe Glu Asp Phe Asp Tyr
        835                 840                 845

Ser Ser Trp Asp Ala Met Cys Tyr Leu Asp Pro Ser Lys Ala Val Glu
    850                 855                 860

Glu Asp Asp Phe Val Val Gly Phe Trp Asn Pro Ser Glu Glu Asn Cys
865                 870                 875                 880

Gly Val Asp Thr Gly Lys Gln Ser Ile Ser Tyr Asp Leu His Thr Glu
                885                 890                 895

Gln Cys Ile Ala Asp Lys Ser Ile Ala Asp Cys Val Glu Ala Leu Leu
            900                 905                 910

Gly Cys Tyr Leu Thr Ser Cys Gly Glu Arg Ala Ala Gln Leu Phe Leu
        915                 920                 925

Cys Ser Leu Gly Leu Lys Val Leu Pro Val Ile Lys Arg Thr Asp Arg
    930                 935                 940

Glu Lys Ala Leu Cys Pro Thr Arg Glu Asn Phe Asn Ser Gln Gln Lys
945                 950                 955                 960

Asn Leu Ser Val Ser Cys Ala Ala Ala Ser Val Ala Ser Ser Arg Ser
                965                 970                 975

Ser Val Leu Lys Asp Ser Glu Tyr Gly Cys Leu Lys Ile Pro Pro Arg
            980                 985                 990

Cys Met Phe Asp His Pro Asp Ala  Asp Lys Thr Leu Asn  His Leu Ile
        995                 1000                 1005

Ser Gly  Phe Glu Asn Phe Glu  Lys Lys Ile Asn Tyr  Arg Phe Lys
    1010                 1015                 1020

Asn Lys  Ala Tyr Leu Leu Gln  Ala Phe Thr His Ala  Ser Tyr His
    1025                 1030                 1035

Tyr Asn  Thr Ile Thr Asp Cys  Tyr Gln Arg Leu Glu  Phe Leu Gly
    1040                 1045                 1050

Asp Ala  Ile Leu Asp Tyr Leu  Ile Thr Lys His Leu  Tyr Glu Asp
    1055                 1060                 1065

Pro Arg  Gln His Ser Pro Gly  Val Leu Thr Asp Leu  Arg Ser Ala
    1070                 1075                 1080

Leu Val  Asn Asn Thr Ile Phe  Ala Ser Leu Ala Val  Lys Tyr Asp
    1085                 1090                 1095

Tyr His  Lys Tyr Phe Lys Ala  Val Ser Pro Glu Leu  Phe His Val
    1100                 1105                 1110

Ile Asp  Asp Phe Val Gln Phe  Gln Leu Glu Lys Asn  Glu Met Gln
    1115                 1120                 1125

Gly Met  Asp Ser Glu Leu Arg  Arg Ser Glu Glu Asp  Glu Glu Lys
    1130                 1135                 1140

Glu Glu  Asp Ile Glu Val Pro  Lys Ala Met Gly Asp  Ile Phe Glu
    1145                 1150                 1155

Ser Leu  Ala Gly Ala Ile Tyr  Met Asp Ser Gly Met  Ser Leu Glu
    1160                 1165                 1170

Thr Val  Trp Gln Val Tyr Tyr  Pro Met Met Arg Pro  Leu Ile Glu
    1175                 1180                 1185

Lys Phe  Ser Ala Asn Val Pro  Arg Ser Pro Val Arg  Glu Leu Leu
    1190                 1195                 1200

Glu Met Glu Pro Glu Thr Ala Lys Phe Ser Pro Ala Glu Arg Thr
    1205                1210                1215

Tyr Asp Gly Lys Val Arg Val Thr Val Glu Val Val Gly Lys Gly
    1220                1225                1230

Lys Phe Lys Gly Val Gly Arg Ser Tyr Arg Ile Ala Lys Ser Ala
    1235                1240                1245

Ala Ala Arg Arg Ala Leu Arg Ser Leu Lys Ala Asn Gln Pro Gln
    1250                1255                1260

Val Pro Asn Ser
    1265

<210>  19
<211>  3804
<212>  DNA
<213>  Artificial

<220>
<223>  A gene encoding human dicer mutant

<400>  19
atgaatcaca aagtgcatca tcatcatcat catatcgaag gtaggaattc gagctcggta      60

cccgcctcca ttgttggtcc accaatgagc tgtgtacgat tggctgaaag agttgtcgct     120

ctcatttgct gtgagaaact gcacaaaatt ggcgaactgg atgaccattt gatgccagtt     180

gggaaagaga ctgttaaata tgaagaggag cttgatttgc atgatgaaga agagaccagt     240

gttccaggaa gaccaggttc cacgaaacga aggcagtgct acccaaaagc aattccagag     300

tgtttgaggg atagttatcc cagacctgat cagccctgtt acctgtatgt gataggaatg     360

gttttaacta cacctttacc tgatgaactc aactttagaa ggcggaagct ctatcctcct     420

gaagatacca caagatgctt tggaatactg acggccaaac ccatacctca gattccacac     480

tttcctgtgt acacacgctc tggagaggtt accatatcca ttgagttgaa gaagtctggt     540

ttcatgttgt ctctacaaat gcttgagttg attacaagac ttcaccagta tatattctca     600

catattcttc ggcttgaaaa acctgcacta gaatttaaac ctacagacgc tgattcagca     660

tactgtgttc tacctcttaa tgttgttaat gactccagca ctttggatat tgactttaaa     720

ttcatggaag atattgagaa gtctgaagct cgcataggca ttcccagtac aaagtataca     780

aaagaaacac cctttgtttt taaattagaa gattaccaag atgccgttat cattccaaga     840

tatcgcaatt ttgatcagcc tcatcgattt tatgtagctg atgtgtacac tgatcttacc     900

ccactcagta aatttccttc ccctgagtat gaaacttttg cagaatatta taaaacaaag     960

tacaaccttg acctaaccaa tctcaaccag ccactgctgg atgtggacca cacatcttca    1020

agacttaatc ttttgacacc tcgacatttg aatcagaagg ggaaagcgct tcctttaagc    1080

agtgctgaga agaggaaagc caaatgggaa agtctgcaga ataaacagat actggttcca    1140

gaactctgtg ctatacatcc aattccagca tcactgtgga gaaaagctgt ttgtctcccc 1200
agcatacttt atcgccttca ctgccttttg actgcagagg agctaagagc ccagactgcc 1260
agcgatgctg gcgtgggagt cagatcactt cctgcggatt ttagataccc taacttagac 1320
ttcgggtgga aaaaatctat tgacagcaaa tctttcatct caatttctaa ctcctcttca 1380
gctgaaaatg ataattactg taagcacagc acaattgtcc ctgaaaatgc tgcacatcaa 1440
ggtgctaata gaacctcctc tctagaaaat catgaccaaa tgtctgtgaa ctgcagaacg 1500
ttgctcagcg agtcccctgg taagctccac gttgaagttt cagcagatct tacagcaatt 1560
aatggtcttt cttacaatca aaatctcgcc aatggcagtt atgatttagc taacagagac 1620
ttttgccaag gaaatcagct aaattactac aagcaggaaa tacccgtgca accaactacc 1680
tcatattcca ttcagaattt atacagttac gagaaccagc cccagcccag cgatgaatgt 1740
actctcctga gtaataaata ccttgatgga aatgctaaca aatctacctc agatggaagt 1800
cctgtgatgg ccgtaatgcc tggtacgaca gacactattc aagtgctcaa gggcaggatg 1860
gattctgagc agagcccttc tattgggtac tcctcaagga ctcttggccc caatcctgga 1920
cttattcttc aggctttgac tctgtcaaac gctagtgatg gatttaacct ggagcggctt 1980
gaaatgcttg gcgactcctt tttaaagcat gccatcacca catatctatt ttgcacttac 2040
cctgatgcgc atgagggccg cctttcatat atgagaagca aaaaggtcag caactgtaat 2100
ctgtatcgcc ttggaaaaaa gaagggacta cccagccgca tggtggtgtc aatatttgat 2160
ccccctgtga attggcttcc tcctggttat gtagtaaatc aagacaaaag caacacagat 2220
aaatgggaaa aagatgaaat gacaaaagac tgcatgctgg cgaatggcaa actggatgag 2280
gattacgagg aggaggatga ggaggaggag agcctgatgt ggagggctcc gaaggaagag 2340
gctgactatg aagatgattt cctggagtat gatcaggaac atatcagatt tatagataat 2400
atgttaatgg ggtcaggagc ttttgtaaag aaaatctctc tttctccttt ttcaaccact 2460
gattctgcat atgaatggaa aatgcccaaa aaatcctcct taggtagtat gccattttca 2520
tcagattttg aggattttga ctacagctct tgggatgcaa tgtgctatct ggatcctagc 2580
aaagctgttg aagaagatga ctttgtggtg gggttctgga atccatcaga agaaaactgt 2640
ggtgttgaca cgggaaagca gtccatttct tacgacttgc acactgagca gtgtattgct 2700
gacaaaagca tagcggactg tgtggaagcc ctgctgggct gctatttaac cagctgtggg 2760
gagagggctg ctcagctttt cctctgttca ctggggctga aggtgctccc ggtaattaaa 2820
aggactgatc gggaaaaggc cctgtgccct actcgggaga atttcaacag ccaacaaaag 2880
aacctttcag tgagctgtgc tgctgcttct gtggccagtt cacgctcttc tgtattgaaa 2940
gactcggaat atggttgttt gaagattcca ccaagatgta tgtttgatca tccagatgca 3000
gataaaacac tgaatcacct tatatcgggg tttgaaaatt ttgaaaagaa aatcaactac 3060
agattcaaga ataaggctta ccttctccag gcttttacac atgcctccta ccactacaat 3120
actatcactg attgttacca gcgcttagaa ttcctgggag atgcgatttt ggactacctc 3180

ataaccaagc acctttatga agacccgcgg cagcactccc cgggggtcct gacagacctg 3240

cggtctgccc tggtcaacaa caccatcttt gcatcgctgg ctgtaaagta cgactaccac 3300

aagtacttca aagctgtctc tcctgagctc ttccatgtca ttgatgactt tgtgcagttt 3360

cagcttgaga agaatgaaat gcaaggaatg gattctgagc ttaggagatc tgaggaggat 3420

gaagagaaag aagaggatat tgaagttcca aaggccatgg gggatatttt tgagtcgctt 3480

gctggtgcca tttacatgga tagtgggatg tcactggaga cagtctggca ggtgtactat 3540

cccatgatgc ggccactaat agaaaagttt tctgcaaatg taccccgttc ccctgtgcga 3600

gaattgcttg aaatggaacc agaaactgcc aaatttagcc cggctgagag aacttacgac 3660

gggaaggtca gagtcactgt ggaagtagta ggaaagggga aatttaaagg tgttggtcga 3720

agttacagga ttgccaaatc tgcagcagca agaagagccc tccgaagcct caaagctaat 3780

caacctcagg ttcccaatag ctaa 3804

<210> 20
<211> 20
<212> DNA
<213> Artificial

<220>
<223> Synthetic primer rsGFP-F to amplify a gene encoding rsGFP

<400> 20
gccacaacat tgaagatgga 20

<210> 21
<211> 20
<212> DNA
<213> Artificial

<220>
<223> Synthetic primer rsGFP-R to amplify a gene encoding rsGFP

<400> 21
gaaagggcag attgtgtgga 20

<210> 22
<211> 20
<212> DNA
<213> Artificial

<220>
<223> Synthetic primer Neo-F to amplify a gene encoding Neo

<400> 22
atagcgttgg ctacccgtga 20

<210> 23
<211> 20
<212> DNA
<213> Artificial

<220>
<223> Synthetic primer Neo-R to amplify a gene encoding Neo

<400> 23

gaaggcgata gaaggcgatg 20

<210> 24
<211> 42
<212> DNA
<213> Artificial

<220>
<223> Synthetic primer dsl-1 to amplify a gene encoding luciferase

<400> 24
gggtaatacg actcactata gggagaatgg aagacgccaa aa 42

<210> 25
<211> 42
<212> DNA
<213> Artificial

<220>
<223> Synthetic primer dsl-2 to amplify a gene encoding luciferase

<400> 25
gggtaatacg actcactata gggagagaac gtgtacatcg ac 42

<210> 26
<211> 34
<212> DNA
<213> Artificial

<220>
<223> Synthetic primer E to amplify a gene encoding CspB

<400> 26
gagcggataa catatgagag gaaaggttaa gtgg 34

<210> 27
<211> 37
<212> DNA
<213> Artificial

<220>
<223> Synthetic primer F to amplify a gene encoding CspB

<400> 27
gagcggataa ggatccttac tcaactactt tcacgtg 37